# EUROPÄISCHE PATENTSCHRIFT

(11) **EP 1 137 810 B1**
(45) Veröffentlichungstag und Bekanntmachung des Hinweises auf die Patenterteilung: **07.05.2008**
(21) Anmeldenummer: 99963338.1
(22) Anmeldetag: 27.11.1999
(51) Int. Cl.: C12Q 1/68, C07K 14/47, C12N 15/12, C12N 15/10, C12N 5/10, C12N 15/81, C12N 15/83, G01N 33/68

(54) **PEPTID SCREENING TEST ZUM NACHWEIS VON GAMMA-SEKRETASE AKTIVITÄT**
PEPTIDE SCREENING ASSAY FOR THE DETERMINATION OF GAMMA-SECRETASE ACTIVITY
ANALYSE SELECTIVE PEPTIDIQUES POUR DÉTECTER L'ACTIVITÉ DE GAMMA-SÉCRÉTASE

(30) Priorität: 07.12.1998 DE 19856261
(43) Veröffentlichungstag der Anmeldung: 04.10.2001
(73) Patentinhaber: Sanofi-Aventis Deutschland GmbH, 65929 Frankfurt am Main (DE)
(72) Erfinder: PERAUS, Gisela, D-80687 München (DE)
(86) Internationale Anmeldenummer: PCT/EP1999/009234
(87) Internationale Veröffentlichungsnummer: WO 2000/034511

(56) Entgegenhaltungen:
- EP-A- 0 653 154
- EP-A- 0 801 307
- WO-A-94/28412
- WO-A-98/15828
- DE-A- 19 849 073
- US-A- 5 667 992
- LINK C D: "TRANSGENIC CAENORHABDITIS ELEGANS AS MODEL SYSTEM TO STUDY AMYLOID FORMATION AND TOXICITY" NEUROBIOLOGY OF AGING,US,TARRYTOWN, NY, Bd. 15, Nr. SUPPL. 01, 29. Juli 1994 (1994-07-29), Seite S33 XP002065640 ISSN: 0197-4580
- WOLFE M S ET AL.: "A substrate-based difluoro ketone selectively inhibits Alzheimer's gamma-secretase activity" JOURNAL OF MEDICINAL CHEMISTRY, Bd. 41, 1998, Seiten 6-9, XP000938942
- URMONEIT B ET AL: "Cationic Lipids (Lipofectamine) and Disturbance of Cellular Cholesterol and Sphingomyelin Distribution Modulates Gamma-Secretase Activity Within Amyloid Precursor Protein In Vitro - An index of oxidative stress" PROSTAGLANDINS AND OTHER LIPID MEDIATORS,US,BUTTERWORTH, STONEHAM, MA, Bd. 55, Nr. 5-6, 1. April 1998 (1998-04-01), Seiten 331-343, XP004128238 ISSN: 0090-6980
- CUBITT A B ET AL: "UNDERSTANDING, IMPROVING AND USING GREEN FLUORESCENT PROTEINS" TIBS TRENDS IN BIOCHEMICAL SCIENCES,EN,ELSEVIER PUBLICATION, CAMBRIDGE, Bd. 20, 1. November 1995 (1995-11-01), Seiten 448-455, XP000606919 ISSN: 0968-0004
- SADOWSKI I ET AL.: "GAL4-VP16 is an unusually potent transcriptional activator" NATURE, Bd. 335, 1988, Seiten 563-564, XP002147306 in der Anmeldung erwähnt
- HIGAKI J ET AL.: "Processing of beta-amyloid precursor protein by cathepsin D" THE JOURNAL OF BIOLOGICAL CHEMISTRY, Bd. 271, Nr. 50, 1996, Seiten 31885-31893, XP002147307
- LICHTENTHALER S F ET AL.: "A novel substrate for analyzing Alzheimer's disease gamma-secretase" FEBS LETTERS, Bd. 453, 1999, Seiten 288-292, XP000937693
- LI, Q-X ET AL.: "Intracellular accumulation of detergent-soluble amyloidogenic Abeta fragment of Alzheimer's disease precursor protein in the hippocampus of aged transgenic mice" JOURNAL OF NEUROCHEMISTRY, Bd. 72, 1999, Seiten 2479-2487, XP000912279

### SEQUENZPROTOKOLL

<110> Aventis Pharma Deutschland GmbH
<120> Aß-Peptid Screening Assay
<130> 1998/L079
<140> PCT/EP 99/09234
   <141> 1999-11-17
<150> 19856261.6
   <151> 1998-12-07
<160> 13
<170> PatentIn Ver. 2.1
<210> 1
   <211> 24
   <212> PRT
   <213> Homo sapiens
<400> 1
   Gly Ala Ile Ile Gly Leu Met Val Gly Gly Val Val Ile Ala Thr Val
    1               5                  10                  15
   Ile Val Ile Thr Leu Val Met Leu
               20
<210> 2
   <211> 12
   <212> PRT
   <213> Homo sapiens
<400> 2
   Gly Ala Ile Ile Gly Leu Met Val Gly Gly Val Val
   1 5 10
<210> 3
   <211> 9
   <212> PRT
   <213> Homo sapiens
<400> 3
   Val Ile Val Ile Thr Leu Val Met Leu
   1 5
<210> 4
   <211> 100
   <212> PRT
   <213> Homo sapiens
<400> 4
   Leu Asp Ala Glu Phe Arg His Asp Ser Gly Tyr Glu Val His His Gln
   1 5 10 15
   Lys Leu Val Phe Phe Ala Glu Asp Val Gly Ser Asn Lys Gly Ala Ile
   20 25 30
   Ile Gly Leu Met Val Gly Gly Val Val Ile Ala Thr Val Ile Val Ile
   35 40 45
   Thr Leu Val Met Leu Lys Lys Lys Gln Tyr Thr Ser Ile His His Gly
   50 55 60
   Val Val Glu Val Asp Ala Ala Val Thr Pro Glu Glu Arg His Leu Ser
   65 70 75 80
   Lys Met Gln Gln Asn Gly Tyr Glu Asn Pro Thr Tyr Lys Phe Phe Glu
   85 90 95
   Gln Met Gln Asn
   100
<210> 5
   <211> 17
   <212> PRT
   <213> Homo sapiens
<400> 5
   Met Leu Pro Gly Leu Ala Leu Phe Leu Leu Ala Ala Trp Thr Ala Arg
   1               5                   10                  15
   Ala
<210> 6
   <211> 117
   <212> PRT
   <213> Homo sapiens
<400> 6
   Met Leu Pro Gly Leu Ala Leu Phe Leu Leu Ala Ala Trp Thr Ala Arg
   1               5                   10                  15
   Ala Leu Asp Ala Glu Phe Arg His Asp Ser Gly Tyr Glu Val His His
               20                  25                  30
   Gln Lys Leu Val Phe Phe Ala Glu Asp Val Gly Ser Asn Lys Gly Ala
           35                  40                  45
   Ile Ile Gly Leu Met Val Gly Gly Val Val Ile Ala Thr Val Ile Val
       50                  55                  60
   Ile Thr Leu Val Met Leu Lys Lys Lys Gln Tyr Thr Ser Ile His His
   65                  70                  75                  80
   Gly Val Val Glu Val Asp Ala Ala Val Thr Pro Glu Glu Arg His Leu
                   85                  90                  95
   Ser Lys Met Gln Gln Asn Gly Tyr Glu Asn Pro Thr Tyr Lys Phe Phe
               100                 105                 110
   Glu Gln Met Gln Asn
           115
<210> 7
   <211> 232
   <212> PRT
   <213> Künstliche Sequenz
<220>
   <223> Beschreibung der künstlichen Sequenz: Mutagen
<400> 7
   Met Lys Leu Leu Ser Ser Ile Glu Gln Ala Cys Asp Ile Cys Arg Leu
     1               5                  10                  15
   Lys Lys Leu Lys Cys Ser Lys Glu Lys Pro Lys Cys Ala Lys Cys Leu
                20                  25                  30
   Lys Asn Asn Trp Glu Cys Arg Tyr Ser Pro Lys Thr Lys Arg Ser Pro
            35                  40                  45
   Leu Thr Arg Ala His Leu Thr Glu Val Glu Ser Arg Leu Glu Arg Leu
       50                  55                  60
   Glu Gln Leu Phe Leu Leu Ile Phe Pro Arg Glu Asp Leu Asp Met Ile
    65                  70                  75                  80
   Leu Lys Met Asp Ser Leu Gln Asp Ile Lys Ala Leu Leu Thr Gly Leu
                    85                  90                  95
   Phe Val Gln Asp Asn Val Asn Lys Asp Ala Val Thr Asp Arg Leu Ala
               100                 105                 110
   Ser Val Glu Thr Asp Met Pro Leu Thr Leu Arg Gln His Arg Ile Ser
           115                 120                 125
   Ala Thr Ser Ser Ser Glu Glu Ser Ser Asn Lys Gly Gln Arg Gln Leu
       130                 135                 140
   Thr Val Ser Pro Glu Phe Pro Gly Ile Trp Ala Pro Pro Thr Asp Val
   145                 150                 155                 160
   Ser Leu Gly Asp Glu Leu His Leu Asp Gly Glu Asp Val Ala Met Ala
                   165                 170                 175
   His Ala Asp Ala Leu Asp Asp Phe Asp Leu Asp Met Leu Gly Asp Gly
               180                 185                 190
   Asp Ser Pro Gly Pro Gly Phe Thr Pro His Asp Ser Ala Pro Tyr Gly
           195                 200                 205
   Ala Leu Asp Met Ala Asp Phe Glu Phe Glu Gln Met Phe Thr Asp Ala
       210                 215                 220
   Leu Gly Ile Asp Glu Tyr Gly Gly
   225                 230
<210> 8
   <211> 1813
   <212> DNA
   <213> Künstliche Sequenz
<220>
   <223> Beschreibung der künstlichen Sequenz: Mutagen
<400> 8
   ggcaaggctt gaccgacaat tgcatgaaga atctgcttag ggttaggcgt tttgcgctgc 60
   ttcgcgatgt acgggccaga tatacgcgtt gacattgatt attgactagt tattaatagt 120
   aatcaattac ggggtcatta gttcatagcc catatatgga gttccgcgtt acataactta 180
   cggtaaatgg cccgcctggc tgaccgccca acgacccccg cccattgacg tcaataatga 240
   cgtatgttcc catagtaacg ccaataggga ctttccattg acgtcaatgg gtggactatt 300
   tacggtaaac tgcccacttg gcagtacatc aagtgtatca tatgccaagt acgcccccta 360
   ttgacgtcaa tgacggtaaa tggcccgcct ggcattatgc ccagtacatg accttatggg 420
   actttcctac ttggcagtac atctacgtat tagtcatcgc tattaccatg gtgatgcggt 480
   tttggcagta catcaatggg cgtggatagc ggtttgactc acggggattt ccaagtctcc 540
   accccattga cgtcaatggg agtttgtttt ggcaccgcgt gtacggtggg aggtctatat 600
   aagcagagct ctctggctaa ctagagaacc cactgcttac tggcttatcg aaattaatac 660
   gactcactat agggagaccc aagctggcta gcgtttaaac ttaagcttca cagctagcgc 720
   actcggtgcc ccgcgcaggg tcgcgatgct gcccggtttg gcactgttcc tgctggccgc 780
   ctggacggct cgggcgctgg atgcagaatt ccgacatgac tcaggatatg aagttcatca 840
   tcaaaaattg gtgttctttg cagaagatgt gggttcaaac aaaggtgcaa tcattggact 900
   catggtgggc ggtgttgtca tagcgacagt gatcgtcatc accttggtga tgctgaagaa 960
   gaaacagtac acatccattc atcatggtgt ggtggaggtt gacgccgctg tcaccccaga 1020
   ggagcgccac ctgtccaaga tgcagcagaa cggctacgaa aatccaacct acaagttctt 1080
   tgagcagatg cagaacgcgc ggggtacccc ggcgatgaag ctactgtctt ctatcgaaca 1140
   agcatgcgat atttgccgac ttaaaaagct caagtgctcc aaagaaaaac cgaagtgcgc 1200
   caagtgtctg aagaacaact gggagtgtcg ctactctccc aaaaccaaaa ggtctccgct 1260
   gactagggca catctgacag aagtggaatc aaggctagaa agactggaac agctatttct 1320
   actgattttt cctcgagaag accttgacat gattttgaaa atggattctt tacaggatat 1380
   aaaagcattg ttaacaggat tatttgtaca agataatgtg aataaagatg ccgtcacaga 1440
   tagattggct tcagtggaga ctgatatgcc tctaacattg agacagcata gaataagtgc 1500
   gacatcatca tcggaagaga gtagtaacaa aggtcaaaga cagttgactg tatcgccgga 1560
   attcccgggg atctgggccc ccccgaccga tgtcagcctg ggggacgagc tccacttaga 1620
   cggcgaggac gtggcgatgg cgcatgccga cgcgctagac gatttcgatc tggacatgtt 1680
   gggggacggg gattccccgg ggccgggatt taccccccac gactccgccc cctacggcgc 1740
   tctggatatg gccgacttcg agtttgagca gatgtttacc gatgcccttg gaattgacga 1800
   gtacggtggg tag                                                    1813
<210> 9
   <211> 5432
   <212> DNA
   <213> Künstliche Sequenz
<220>
   <223> Beschreibung der künstlichen Sequenz: Mutagen
<400> 9
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagcttggta ccgagctcgg atccactagt ccagtgtggt ggaattctgc 960
   agatatccag cacagtggcg gccgctcgag tctagagggc ccgtttaaac ccgctgatca 1020
   gcctcgactg tgccttctag ttgccagcca tctgttgttt gcccctcccc cgtgccttcc 1080
   ttgaccctgg aaggtgccac tcccactgtc ctttcctaat aaaatgagga aattgcatcg 1140
   cattgtctga gtaggtgtca ttctattctg gggggtgggg tggggcagga cagcaagggg 1200
   gaggattggg aagacaatag caggcatgct ggggatgcgg tgggctctat ggcttctgag 1260
   gcggaaagaa ccagctgggg ctctaggggg tatccccacg cgccctgtag cggcgcatta 1320
   agcgcggcgg gtgtggtggt tacgcgcagc gtgaccgcta cacttgccag cgccctagcg 1380
   cccgctcctt tcgctttctt cccttccttt ctcgccacgt tcgccggctt tccccgtcaa 1440
   gctctaaatc ggggcatccc tttagggttc cgatttagtg ctttacggca cctcgacccc 1500
   aaaaaacttg attagggtga tggttcacgt agtgggccat cgccctgata gacggttttt 1560
   cgccctttga cgttggagtc cacgttcttt aatagtggac tcttgttcca aactggaaca 1620
   acactcaacc ctatctcggt ctattctttt gatttataag ggattttggg gatttcggcc 1680
   tattggttaa aaaatgagct gatttaacaa aaatttaacg cgaattaatt ctgtggaatg 1740
   tgtgtcagtt agggtgtgga aagtccccag gctccccagg caggcagaag tatgcaaagc 1800
   atgcatctca attagtcagc aaccaggtgt ggaaagtccc caggctcccc agcaggcaga 1860
   agtatgcaaa gcatgcatct caattagtca gcaaccatag tcccgcccct aactccgccc 1920
   atcccgcccc taactccgcc cagttccgcc cattctccgc cccatggctg actaattttt 1980
   tttatttatg cagaggccga ggccgcctct gcctctgagc tattccagaa gtagtgagga 2040
   ggcttttttg gaggcctagg cttttgcaaa aagctcccgg gagcttgtat atccattttc 2100
   ggatctgatc aagagacagg atgaggatcg tttcgcatga ttgaacaaga tggattgcac 2160
   gcaggttctc cggccgcttg ggtggagagg ctattcggct atgactgggc acaacagaca 2220
   atcggctgct ctgatgccgc cgtgttccgg ctgtcagcgc aggggcgccc ggttcttttt 2280
   gtcaagaccg acctgtccgg tgccctgaat gaactgcagg acgaggcagc gcggctatcg 2340
   tggctggcca cgacgggcgt tccttgcgca gctgtgctcg acgttgtcac tgaagcggga 2400
   agggactggc tgctattggg cgaagtgccg gggcaggatc tcctgtcatc tcaccttgct 2460
   cctgccgaga aagtatccat catggctgat gcaatgcggc ggctgcatac gcttgatccg 2520
   gctacctgcc cattcgacca ccaagcgaaa catcgcatcg agcgagcacg tactcggatg 2580
   gaagccggtc ttgtcgatca ggatgatctg gacgaagagc atcaggggct cgcgccagcc 2640
   gaactgttcg ccaggctcaa ggcgcgcatg cccgacggcg aggatctcgt cgtgacccat 2700
   ggcgatgcct gcttgccgaa tatcatggtg gaaaatggcc gcttttctgg attcatcgac 2760
   tgtggccggc tgggtgtggc ggaccgctat caggacatag cgttggctac ccgtgatatt 2820
   gctgaagagc ttggcggcga atgggctgac cgcttcctcg tgctttacgg tatcgccgct 2880
   cccgattcgc agcgcatcgc cttctatcgc cttcttgacg agttcttctg agcgggactc 2940
   tggggttcga aatgaccgac caagcgacgc ccaacctgcc atcacgagat ttcgattcca 3000
   ccgccgcctt ctatgaaagg ttgggcttcg gaatcgtttt ccgggacgcc ggctggatga 3060
   tcctccagcg cggggatctc atgctggagt tcttcgccca ccccaacttg tttattgcag 3120
   cttataatgg ttacaaataa agcaatagca tcacaaattt cacaaataaa gcattttttt 3180
   cactgcattc tagttgtggt ttgtccaaac tcatcaatgt atcttatcat gtctgtatac 3240
   cgtcgacctc tagctagagc ttggcgtaat catggtcata gctgtttcct gtgtgaaatt 3300
   gttatccgct cacaattcca cacaacatac gagccggaag cataaagtgt aaagcctggg 3360
   gtgcctaatg agtgagctaa ctcacattaa ttgcgttgcg ctcactgccc gctttccagt 3420
   cgggaaacct gtcgtgccag ctgcattaat gaatcggcca acgcgcgggg agaggcggtt 3480
   tgcgtattgg gcgctcttcc gcttcctcgc tcactgactc gctgcgctcg gtcgttcggc 3540
   tgcggcgagc ggtatcagct cactcaaagg cggtaatacg gttatccaca gaatcagggg 3600
   ataacgcagg aaagaacatg tgagcaaaag gccagcaaaa ggccaggaac cgtaaaaagg 3660
   ccgcgttgct ggcgtttttc cataggctcc gcccccctga cgagcatcac aaaaatcgac 3720
   gctcaagtca gaggtggcga aacccgacag gactataaag ataccaggcg tttccccctg 3780
   gaagctccct cgtgcgctct cctgttccga ccctgccgct taccggatac ctgtccgcct 3840
   ttctcccttc gggaagcgtg gcgctttctc aatgctcacg ctgtaggtat ctcagttcgg 3900
   tgtaggtcgt tcgctccaag ctgggctgtg tgcacgaacc ccccgttcag cccgaccgct 3960
   gcgccttatc cggtaactat cgtcttgagt ccaacccggt aagacacgac ttatcgccac 4020
   tggcagcagc cactggtaac aggattagca gagcgaggta tgtaggcggt gctacagagt 4080
   tcttgaagtg gtggcctaac tacggctaca ctagaaggac agtatttggt atctgcgctc 4140
   tgctgaagcc agttaccttc ggaaaaagag ttggtagctc ttgatccggc aaacaaacca 4200
   ccgctggtag cggtggtttt tttgtttgca agcagcagat tacgcgcaga aaaaaaggat 4260
   ctcaagaaga tcctttgatc ttttctacgg ggtctgacgc tcagtggaac gaaaactcac 4320
   gttaagggat tttggtcatg agattatcaa aaaggatctt cacctagatc cttttaaatt 4380
   aaaaatgaag ttttaaatca atctaaagta tatatgagta aacttggtct gacagttacc 4440
   aatgcttaat cagtgaggca cctatctcag cgatctgtct atttcgttca tccatagttg 4500
   cctgactccc cgtcgtgtag ataactacga tacgggaggg cttaccatct ggccccagtg 4560
   ctgcaatgat accgcgagac ccacgctcac cggctccaga tttatcagca ataaaccagc 4620
   cagccggaag ggccgagcgc agaagtggtc ctgcaacttt atccgcctcc atccagtcta 4680
   ttaattgttg ccgggaagct agagtaagta gttcgccagt taatagtttg cgcaacgttg 4740
   ttgccattgc tacaggcatc gtggtgtcac gctcgtcgtt tggtatggct tcattcagct 4800
   ccggttccca acgatcaagg cgagttacat gatcccccat gttgtgcaaa aaagcggtta 4860
   gctccttcgg tcctccgatc gttgtcagaa gtaagttggc cgcagtgtta tcactcatgg 4920
   ttatggcagc actgcataat tctcttactg tcatgccatc cgtaagatgc ttttctgtga 4980
   ctggtgagta ctcaaccaag tcattctgag aatagtgtat gcggcgaccg agttgctctt 5040
   gcccggcgtc aatacgggat aataccgcgc cacatagcag aactttaaaa gtgctcatca 5100
   ttggaaaacg ttcttcgggg cgaaaactct caaggatctt accgctgttg agatccagtt 5160
   cgatgtaacc cactcgtgca cccaactgat cttcagcatc ttttactttc accagcgttt 5220
   ctgggtgagc aaaaacagga aggcaaaatg ccgcaaaaaa gggaataagg gcgacacgga 5280
   aatgttgaat actcatactc ttcctttttc aatattattg aagcatttat cagggttatt 5340
   gtctcatgag cggatacata tttgaatgta tttagaaaaa taaacaaata ggggttccgc 5400
   gcacatttcc ccgaaaagtg ccacctgacg tc                               5432
<210> 10
   <211> 3354
   <212> DNA
   <213> Homo sapiens
<400> 10
   agtttcctcg gcagcggtag gcgagagcac gcggaggagc gtgcgcgggg gccccgggag 60
   acggcggcgg tggcggcgcg ggcagagcaa ggacgcggcg gatcccactc gcacagcagc 120
   gcactcggtg ccccgcgcag ggtcgcgatg ctgcccggtt tggcactgct cctgctggcc 180
   gcctggacgg ctcgggcgct ggaggtaccc actgatggta atgctggcct gctggctgaa 240
   ccccagattg ccatgttctg tggcagactg aacatgcaca tgaatgtcca gaatgggaag 300
   tgggattcag atccatcagg gaccaaaacc tgcattgata ccaaggaagg catcctgcag 360
   tattgccaag aagtctaccc tgaactgcag atcaccaatg tggtagaagc caaccaacca 420
   gtgaccatcc agaactggtg caagcggggc cgcaagcagt gcaagaccca tccccacttt 480
   gtgattccct accgctgctt agttggtgag tttgtaagtg atgcccttct cgttcctgac 540
   aagtgcaaat tcttacacca ggagaggatg gatgtttgcg aaactcatct tcactggcac 600
   accgtcgcca aagagacatg cagtgagaag agtaccaact tgcatgacta cggcatgttg 660
   ctgccctgcg gaattgacaa gttccgaggg gtagagtttg tgtgttgccc actggctgaa 720
   gaaagtgaca atgtggattc tgctgatgcg gaggaggatg actcggatgt ctggtggggc 780
   ggagcagaca cagactatgc agatgggagt gaagacaaag tagtagaagt agcagaggag 840
   gaagaagtgg ctgaggtgga agaagaagaa gccgatgatg acgaggacga tgaggatggt 900
   gatgaggtag aggaagaggc tgaggaaccc tacgaagaag ccacagagag aaccaccagc 960
   attgccacca ccaccaccac caccacagag tctgtggaag aggtggttcg agttcctaca 1020
   acagcagcca gtacccctga tgccgttgac aagtatctcg agacacctgg ggatgagaat 1080
   gaacatgccc atttccagaa agccaaagag aggcttgagg ccaagcaccg agagagaatg 1140
   tcccaggtca tgagagaatg ggaagaggca gaacgtcaag caaagaactt gcctaaagct 1200
   gataagaagg cagttatcca gcatttccag gagaaagtgg aatctttgga acaggaagca 1260
   gccaacgaga gacagcagct ggtggagaca cacatggcca gagtggaagc catgctcaat 1320
   gaccgccgcc gcctggccct ggagaactac atcaccgctc tgcaggctgt tcctcctcgg 1380
   cctcgtcacg tgttcaatat gctaaagaag tatgtccgcg cagaacagaa ggacagacag 1440
   cacaccctaa agcatttcga gcatgtgcgc atggtggatc ccaagaaagc cgctcagatc 1500
   cggtcccagg ttatgacaca cctccgtgtg atttatgagc gcatgaatca gtctctctcc 1560
   ctgctctaca acgtgcctgc agtggccgag gagattcagg atgaagttga tgagctgctt 1620
   cagaaagagc aaaactattc agatgacgtc ttggccaaca tgattagtga accaaggatc 1680
   agttacggaa acgatgctct catgccatct ttgaccgaaa cgaaaaccac cgtggagctc 1740
   cttcccgtga atggagagtt cagcctggac gatctccagc cgtggcattc ttttggggct 1800
   gactctgtgc cagccaacac agaaaacgaa gttgagcctg ttgatgcccg ccctgctgcc 1860
   gaccgaggac tgaccactcg accaggttct gggttgacaa atatcaagac ggaggagatc 1920
   tctgaagtga agatggatgc agaattccga catgactcag gatatgaagt tcatcatcaa 1980
   aaattggtgt tctttgcaga agatgtgggt tcaaacaaag gtgcaatcat tggactcatg 2040
   gtgggcggtg ttgtcatagc gacagtgatc gtcatcacct tggtgatgct gaagaagaaa 2100
   cagtacacat ccattcatca tggtgtggtg gaggttgacg ccgctgtcac cccagaggag 2160
   cgccacctgt ccaagatgca gcagaacggc tacgaaaatc caacctacaa gttctttgag 2220
   cagatgcaga actagacccc cgccacagca gcctctgaag ttggacagca aaaccattgc 2280
   ttcactaccc atcggtgtcc atttatagaa taatgtggga agaaacaaac ccgttttatg 2340
   atttactcat tatcgccttt tgacagctgt gctgtaacac aagtagatgc ctgaacttga 2400
   attaatccac acatcagtaa tgtattctat ctctctttac attttggtct ctatactaca 2460
   ttattaatgg gttttgtgta ctgtaaagaa tttagctgta tcaaactagt gcatgaatag 2520
   attctctcct gattatttat cacatagccc cttagccagt tgtatattat tcttgtggtt 2580
   tgtgacccaa ttaagtccta ctttacatat gctttaagaa tcgatggggg atgcttcatg 2640
   tgaacgtggg agttcagctg cttctcttgc ctaagtattc ctttcctgat cactatgcat 2700
   tttaaagtta aacattttta agtatttcag atgctttaga gagatttttt ttccatgact 2760
   gcattttact gtacagattg ctgcttctgc tatatttgtg atataggaat taagaggata 2820
   cacacgtttg tttcttcgtg cctgttttat gtgcacacat taggcattga gacttcaagc 2880
   ttttcttttt ttgtccacgt atctttgggt ctttgataaa gaaaagaatc cctgttcatt 2940
   gtaagcactt ttacggggcg ggtggggagg ggtgctctgc tggtcttcaa ttaccaagaa 3000
   ttctccaaaa caattttctg caggatgatt gtacagaatc attgcttatg acatgatcgc 3060
   tttctacact gtattacata aataaattaa ataaaataac cccgggcaag acttttcttt 3120
   gaaggatgac tacagacatt aaataatcga agtaattttg ggtggggaga agaggcagat 3180
   tcaattttct ttaaccagtc tgaagtttca tttatgatac aaaagaagat gaaaatggaa 3240
   gtggcaatat aaggggatga ggaaggcatg cctggacaaa cccttctttt aagatgtgtc 3300
   ttcaatttgt ataaaatggt gttttcatgt aaataaatac attcttggag gagc       3354
<210> 11
   <211> 8667
   <212> DNA
   <213> Künstliche Sequenz
<220>
   <223> Beschreibung der künstlichen Sequenz: Mutagen
<400> 11
   gacgaaaggg cctcgtgata cgcctatttt tataggttaa tgtcatgata ataatggttt 60
   cttaggacgg atcgcttgcc tgtaacttac acgcgcctcg tatcttttaa tgatggaata 120
   atttgggaat ttactctgtg tttatttatt tttatgtttt gtatttggat tttagaaagt 180
   aaataaagaa ggtagaagag ttacggaatg aagaaaaaaa aataaacaaa ggtttaaaaa 240
   atttcaacaa aaagcgtact ttacatatat atttattaga caagaaaagc agattaaata 300
   gatatacatt cgattaacga taagtaaaat gtaaaatcac aggattttcg tgtgtggtct 360
   tctacacaga caagatgaaa caattcggca ttaatacctg agagcaggaa gagcaagata 420
   aaaggtagta tttgttggcg atccccctag agtcttttac atcttcggaa aacaaaaact 480
   attttttctt taatttcttt ttttactttc tatttttaat ttatatattt atattaaaaa 540
   atttaaatta taattatttt tatagcacgt gatgaaaagg acccaggtgg cacttttcgg 600
   ggaaatgtgc gcggaacccc tatttgttta tttttctaaa tacattcaaa tatgtatccg 660
   ctcatgagac aataaccctg ataaatgctt caataatctg cagctctggc ccgtgtctca 720
   aaatctctga tgttacattg cacaagataa aaatatatca tcatgaacaa taaaactgtc 780
   tgcttacata aacagtaata caaggggtgt tatgagccat attcaacggg aaacgtcttg 840
   ctggaggccg cgattaaatt ccaacatgga tgctgattta tatgggtata aatgggctcg 900
   cgataatgtc gggcaatcag gtgcgacaat ctttcgattg tatgggaagc ccgatgcgcc 960
   agagttgttt ctgaaacatg gcaaaggtag cgttgccaat gatgttacag atgagatggt 1020
   cagactaaac tggctgacgg aatttatgcc tcttccgacc atcaagcatt ttatccgtac 1080
   tcctgatgat gcatggttac tcaccactgc gatccgcggg aaaacagcat tccaggtatt 1140
   agaagaatat cctgattcag gtgaaaatat tgttgatgcg ctggcagtgt tcctgcgccg 1200
   gttgcattcg attcctgttt gtaattgtcc ttttaacagc gatcgcgtat ttcgtctcgc 1260
   tcaggcgcaa tcacgaatga ataacggttt ggttgatgcg agtgattttg atgacgagcg 1320
   taatggctgg cctgttgaac aagtctggaa agaaatgcat acgcttttgc cattctcacc 1380
   ggattcagtc gtcactcatg gtgatttctc acttgataac cttatttttg acgaggggaa 1440
   attaataggt tgtattgatg ttggacgagt cggaatcgca gaccgatacc aggatcttgc 1500
   catcctatgg aactgcctcg gtgagttttc tccttcatta cagaaacggc tttttcaaaa 1560
   atatggtatt gataatcctg atatgaataa attgcagttt catttgatgc tcgatgagtt 1620
   tttctaatca gaattggtta attggttgta acactggcag agcattacgc tgacttgacg 1680
   ggacggcgca tgaccaaaat cccttaacgt gagttttcgt tccactgagc gtcagacccc 1740
   gtagaaaaga tcaaaggatc ttcttgagat cctttttttc tgcgcgtaat ctgctgcttg 1800
   caaacaaaaa aaccaccgct accagcggtg gtttgtttgc cggatcaaga gctaccaact 1860
   ctttttccga aggtaactgg cttcagcaga gcgcagatac caaatactgt ccttctagtg 1920
   tagccgtagt taggccacca cttcaagaac tctgtagcac cgcctacata cctcgctctg 1980
   ctaatcctgt taccagtggc tgctgccagt ggcgataagt cgtgtcttac cgggttggac 2040
   tcaagacgat agttaccgga taaggcgcag cggtcgggct gaacgggggg ttcgtgcaca 2100
   cagcccagct tggagcgaac gacctacacc gaactgagat acctacagcg tgagcattga 2160
   gaaagcgcca cgcttcccga agggagaaag gcggacaggt atccggtaag cggcagggtc 2220
   ggaacaggag agcgcacgag ggagcttcca gggggaaacg cctggtatct ttatagtcct 2280
   gtcgggtttc gccacctctg acttgagcgt cgatttttgt gatgctcgtc aggggggcgg 2340
   agcctatgga aaaacgccag caacgcggcc tttttacggt tcctggcctt ttgctggcct 2400
   tttgctcaca tgttctttcc tgcgttatcc cctgattctg tggataaccg tattaccgcc 2460
   tttgagtgag ctgataccgc tcgccgcagc cgaacgaccg agcgcagcga gtcagtgagc 2520
   gaggaagcgg aagagcgccc aatacgcaaa ccgcctctcc ccgcgcgttg gccgattcat 2580
   taatgcagct ggcacgacag gtttcccgac tggaaagcgg gcagtgagcg caacgcaatt 2640
   aatgtgagtt acctcactca ttaggcaccc caggctttac actttatgct tccggctcct 2700
   atgttgtgtg gaattgtgag cggataacaa tttcacacag gaaacagcta tgaccatgat 2760
   tacgccaagc tcggaattaa ccctcactaa agggaacaaa agctggtacc gatcccgagc 2820
   tttgcaaatt aaagccttcg agcgtcccaa aaccttctca agcaaggttt tcagtataat 2880
   gttacatgcg tacacgcgtc tgtacagaaa aaaaagaaaa atttgaaata taaataacgt 2940
   tcttaatact aacataacta taaaaaaata aatagggacc tagacttcag gttgtctaac 3000
   tccttccttt tcggttagag cggatgtggg gggagggcgt gaatgtaagc gtgacataac 3060
   taattacatg atatcgacaa aggaaaaggg gcctgtttac tcacaggctt ttttcaagta 3120
   ggtaattaag tcgtttctgt ctttttcctt cttcaaccca ccaaaggcca tcttggtact 3180
   tttttttttt tttttttttt tttttttttt tttttttttt tttttttttt tttttttttt 3240
   tttttttttt tttttttttt tttttttttt tttcatagaa ataatacaga agtagatgtt 3300
   gaattagatt aaactgaaga tatataattt attggaaaat acatagagct ttttgttgat 3360
   gcgcttaagc gatcaattca acaacaccac cagcagctct gattttttct tcagccaact 3420
   tggagacgaa tctagctttg acgataactg gaacatttgg aattctaccc ttacccaaga 3480
   tcttaccgta accggctgcc aaagtgtcaa taactggagc agtttcctta gaagcagatt 3540
   tcaagtattg gtctctcttg tcttctggga tcaatgtcca caatttgtcc aagttcaaga 3600
   ctggcttcca gaaatgagct tgttgcttgt ggaagtatct cataccaacc ttaccgaaat 3660
   aacctggatg gtatttatcc atgttaattc tgtggtgatg ttgaccaccg gccatacctc 3720
   taccaccggg gtgctttctg tgcttaccga tacgaccttt accggctgag acgtgacctc 3780
   tgtgctttct agtcttagtg aatctggaag gcattcttga ttagttggat gattgttctg 3840
   ggatttaatg caaaaatcac ttaagaagga aaatcaacgg agaaagcaaa cgccatctta 3900
   aatatacggg atacagatga aagggtttga acctatctgg aaaatagcat taaacaagcg 3960
   aaaaactgcg aggaaaattg tttgcgtctc tgcgggctat tcacgcgcca gaggaaaata 4020
   ggaaaaataa cagggcatta gaaaaataat tttgattttg gtaatgtgtg ggtcctggtg 4080
   tacagatgtt acattggtta cagtactctt gtttttgctg tgtttttcga tgaatctcca 4140
   aaatggttgt tagcacatgg aagagtcacc gatgctaagt tatctctatg taagctacgt 4200
   ggcgtgactt ttgatgaagc cgcacaagag atacaggatt ggcaactgca aatagaatct 4260
   ggggatcccc cctcgagatc cgggatcgaa gaaatgatgg taaatgaaat aggaaatcaa 4320
   ggagcatgaa ggcaaaagac aaatataagg gtcgaacgaa aaataaagtg aaaagtgttg 4380
   atatgatgta tttggctttg cggcgccgaa aaaacgagtt tacgcaattg cacaatcatg 4440
   ctgactctgt ggcggacccg cgctcttgcc ggcccggcga taacgctggg cgtgaggctg 4500
   tgcccggcgg agttttttgc gcctgcattt tccaaggttt accctgcgct aaggggcgag 4560
   attggagaag caataagaat gccggttggg gttgcgatga tgacgaccac gacaactggt 4620
   gtcattattt aagttgccga aagaacctga gtgcatttgc aacatgagta tactagaaga 4680
   atgagccaag acttgcgaga cgcgagtttg ccggtggtgc gaacaataga gcgaccatga 4740
   ccttgaaggt gagacgcgca taaccgctag agtactttga agaggaaaca gcaatagggt 4800
   tgctaccagt ataaatagac aggtacatac aacactggaa atggttgtct gtttgagtac 4860
   gctttcaatt catttgggtg tgcactttat tatgttacaa tatggaaggg aactttacac 4920
   ttctcctatg cacatatatt aattaaagtc caatgctagt agagaagggg ggtaacaccc 4980
   ctccgcgctc ttttccgatt tttttctaaa ccgtggaata tttcggatat ccttttgttg 5040
   tttccgggtg tacaatatgg acttcctctt ttctggcaac caaacccata catcgggatt 5100
   cctataatac cttcgttggt ctccctaaca tgtaggtggc ggaggggaga tatacaatag 5160
   aacagatacc agacaagaca taatgggcta aacaagacta caccaattac actgcctcat 5220
   tgatggtggt acataacgaa ctaatactgt agccctagac ttgatagcca tcatcatatc 5280
   gaagtttcac tacccttttt ccatttgcca tctattgaag taataatagg cgcatgcaac 5340
   ttcttttctt tttttttctt ttctctctcc cccgttgttg tctcaccata tccgcaatga 5400
   caaaaaaaat gatggaagac actaaaggaa aaaattaacg acaaagacag caccaacaga 5460
   tgtcgttgtt ccagagctga tgaggggtat cttcgaacac acgaaacttt ttccttcctt 5520
   cattcacgca cactactctc taatgagcaa cggtatacgg ccttccttcc agttacttga 5580
   atttgaaata aaaaaagttt gccgctttgc tatcaagtat aaatagacct gcaattatta 5640
   atcttttgtt tcctcgtcat tgttctcgtt ccctttcttc cttgtttctt tttctgcaca 5700
   atatttcaag ctataccaag catacaatca actccaagct tgaagcaagc ctcctgaaag 5760
   atgaagctac tgtcttctat cgaacaagca tgcgatattt gccgacttaa aaagctcaag 5820
   tgctccaaag aaaaaccgaa gtgcgccaag tgtctgaaga acaactggga gtgtcgctac 5880
   tctcccaaaa ccaaaaggtc tccgctgact agggcacatc tgacagaagt ggaatcaagg 5940
   ctagaaagac tggaacagct atttctactg atttttcctc gagaagacct tgacatgatt 6000
   ttgaaaatgg attctttaca ggatataaaa gcattgttaa caggattatt tgtacaagat 6060
   aatgtgaata aagatgccgt cacagataga ttggcttcag tggagactga tatgcctcta 6120
   acattgagac agcatagaat aagtgcgaca tcatcatcgg aagagagtag taacaaaggt 6180
   caaagacagt tgactgtatc gtcgaggtcg accccgggtg ctagcaaggc cttgtggcca 6240
   gccatggcaa ctagtgcggc cgctaagtaa gtaagacgtc gagctctaag taagtaacgg 6300
   ccgccaccgc ggtggagctt tggacttctt cgccagaggt ttggtcaagt ctccaatcaa 6360
   ggttgtcggc ttgtctacct tgccagaaat ttacgaaaag atggaaaagg gtcaaatcgt 6420
   tggtagatac gttgttgaca cttctaaata agcgaatttc ttatgattta tgatttttat 6480
   tattaaataa gttataaaaa aaataagtgt atacaaattt taaagtgact cttaggtttt 6540
   aaaacgaaaa ttcttgttct tgagtaactc tttcctgtag gtcaggttgc tttctcaggt 6600
   atagcatgag gtcgctctta ttgaccacac ctctaccggc atgccgagca aatgcctgca 6660
   aatcgctccc catttcaccc aattgtagat atgctaactc cagcaatgag ttgatgaatc 6720
   tcggtgtgta ttttatgtcc tcagaggaca atacctgttg taatcgttct tccacacgga 6780
   tcccaattcg ccctatagtg agtcgtatta caattcactg gccgtcgttt tacaacgtcg 6840
   tgactgggaa aaccctggcg ttacccaact taatcgcctt gcagcacatc cccctttcgc 6900
   cagctggcgt aatagcgaag aggcccgcac cgatcgccct tcccaacagt tgcgcagcct 6960
   gaatggcgaa tggacgcgcc ctgtagcggc gcattaagcg cggcgggtgt ggtggttacg 7020
   cgcagcgtga ccgctacact tgccagcgcc ctagcgcccg ctcctttcgc tttcttccct 7080
   tcctttctcg ccacgttcgc cggctttccc cgtcaagctc taaatcgggg gctcccttta 7140
   gggttccgat ttagtgcttt acggcacctc gaccccaaaa aacttgatta gggtgatggt 7200
   tcacgtagtg ggccatcgcc ctgatagacg gtttttcgcc ctttgacgtt ggagtccacg 7260
   ttctttaata gtggactctt gttccaaact ggaacaacac tcaaccctat ctcggtctat 7320
   tcttttgatt tataagggat tttgccgatt tcggcctatt ggttaaaaaa tgagctgatt 7380
   taacaaaaat ttaacgcgaa ttttaacaaa atattaacgt ttacaatttc ctgatgcggt 7440
   attttctcct tacgcatctg tgcggtattt cacaccgcag gcaagtgcac aaacaatact 7500
   taaataaata ctactcagta ataacctatt tcttagcatt tttgacgaaa tttgctattt 7560
   tgttagagtc ttttacacca tttgtctcca cacctccgct tacatcaaca ccaataacgc 7620
   catttaatct aagcgcatca ccaacatttt ctggcgtcag tccaccagct aacataaaat 7680
   gtaagctttc ggggctctct tgccttccaa cccagtcaga aatcgagttc caatccaaaa 7740
   gttcacctgt cccacctgct tctgaatcaa acaagggaat aaacgaatga ggtttctgtg 7800
   aagctgcact gagtagtatg ttgcagtctt ttggaaatac gagtctttta ataactggca 7860
   aaccgaggaa ctcttggtat tcttgccacg actcatctcc atgcagttgg acgatatcaa 7920
   tgccgtaatc attgaccaga gccaaaacat cctccttagg ttgattacga aacacgccaa 7980
   ccaagtattt cggagtgcct gaactatttt tatatgcttt tacaagactt gaaattttcc 8040
   ttgcaataac cgggtcaatt gttctctttc tattgggcac acatataata cccagcaagt 8100
   cagcatcgga atctagagca cattctgcgg cctctgtgct ctgcaagccg caaactttca 8160
   ccaatggacc agaactacct gtgaaattaa taacagacat actccaagct gcctttgtgt 8220
   gcttaatcac gtatactcac gtgctcaata gtcaccaatg ccctccctct tggccctctc 8280
   cttttctttt ttcgaccgaa ttaattctta atcggcaaaa aaagaaaagc tccggatcaa 8340
   gattgtacgt aaggtgacaa gctatttttc aataaagaat atcttccact actgccatct 8400
   ggcgtcataa ctgcaaagta cacatatatt acgatgctgt ctattaaatg cttcctatat 8460
   tatatatata gtaatgtcgt ttatggtgca ctctcagtac aatctgctct gatgccgcat 8520
   agttaagcca gccccgacac ccgccaacac ccgctgacgc gccctgacgg gcttgtctgc 8580
   tcccggcatc cgcttacaga caagctgtga ccgtctccgg gagctgcatg tgtcagaggt 8640
   tttcaccgtc atcaccgaaa cgcgcga                                     8667
<210> 12
   <211> 8331
   <212> DNA
   <213> Künstliche Sequenz
<220>
   <223> Beschreibung der künstlichen Sequenz: Mutagen
<400> 12
   acgaaagggc ctcgtgatac gcctattttt ataggttaat gtcatgataa taatggtttc 60
   ttaggacgga tcgcttgcct gtaacttaca cgcgcctcgt atcttttaat gatggaataa 120
   tttgggaatt tactctgtgt ttatttattt ttatgttttg tatttggatt ttagaaagta 180
   aataaagaag gtagaagagt tacggaatga agaaaaaaaa ataaacaaag gtttaaaaaa 240
   tttcaacaaa aagcgtactt tacatatata tttattagac aagaaaagca gattaaatag 300
   atatacattc gattaacgat aagtaaaatg taaaatcaca ggattttcgt gtgtggtctt 360
   ctacacagac aagatgaaac aattcggcat taatacctga gagcaggaag agcaagataa 420
   aaggtagtat ttgttggcga tccccctaga gtcttttaca tcttcggaaa acaaaaacta 480
   tttttctttt aatttctttt tttactttct atttttaatt tatatattta tattaaaaaa 540
   tttaaattat aattattttt atagcacgtg atgaaaagga cccaggtggc acttttcggg 600
   gaaatgtgcg cggaacccct atttgtttat ttttctaaat acattcaaat atgtatccgc 660
   tcatgagaca ataaccctga taaatgcttc aataatctgc agctctggcc cgtgtctcaa 720
   aatctctgat gttacattgc acaagataaa aatatatcat catgaacaat aaaactgtct 780
   gcttacataa acagtaatac aaggggtgtt atgagccata ttcaacggga aacgtcttgc 840
   tggaggccgc gattaaattc caacatggat gctgatttat atgggtataa atgggctcgc 900
   gataatgtcg ggcaatcagg tgcgacaatc tttcgattgt atgggaagcc cgatgcgcca 960
   gagttgtttc tgaaacatgg caaaggtagc gttgccaatg atgttacaga tgagatggtc 1020
   agactaaact ggctgacgga atttatgcct cttccgacca tcaagcattt tatccgtact 1080
   cctgatgatg catggttact caccactgcg atccgcggga aaacagcatt ccaggtatta 1140
   gaagaatatc ctgattcagg tgaaaatatt gttgatgcgc tggcagtgtt cctgcgccgg 1200
   ttgcattcga ttcctgtttg taattgtcct tttaacagcg atcgcgtatt tcgtctcgct 1260
   caggcgcaat cacgaatgaa taacggtttg gttgatgcga gtgattttga tgacgagcgt 1320
   aatggctggc ctgttgaaca agtctggaaa gaaatgcata cgcttttgcc attctcaccg 1380
   gattcagtcg tcactcatgg tgatttctca cttgataacc ttatttttga cgaggggaaa 1440
   ttaataggtt gtattgatgt tggacgagtc ggaatcgcag accgatacca ggatcttgcc 1500
   atcctatgga actgcctcgg tgagttttct ccttcattac agaaacggct ttttcaaaaa 1560
   tatggtattg ataatcctga tatgaataaa ttgcagtttc atttgatgct cgatgagttt 1620
   ttctaatcag aattggttaa ttggttgtaa cactggcaga gcattacgct gacttgacgg 1680
   gacggcgcat gaccaaaatc ccttaacgtg agttttcgtt ccactgagcg tcagaccccg 1740
   tagaaaagat caaaggatct tcttgagatc ctttttttct gcgcgtaatc tgctgcttgc 1800
   aaacaaaaaa accaccgcta ccagcggtgg tttgtttgcc ggatcaagag ctaccaactc 1860
   tttttccgaa ggtaactggc ttcagcagag cgcagatacc aaatactgtc cttctagtgt 1920
   agccgtagtt aggccaccac ttcaagaact ctgtagcacc gcctacatac ctcgctctgc 1980
   taatcctgtt accagtggct gctgccagtg gcgataagtc gtgtcttacc gggttggact 2040
   caagacgata gttaccggat aaggcgcagc ggtcgggctg aacggggggt tcgtgcacac 2100
   agcccagctt ggagcgaacg acctacaccg aactgagata cctacagcgt gagcattgag 2160
   aaagcgccac gcttcccgaa gggagaaagg cggacaggta tccggtaagc ggcagggtcg 2220
   gaacaggaga gcgcacgagg gagcttccag ggggaaacgc ctggtatctt tatagtcctg 2280
   tcgggtttcg ccacctctga cttgagcgtc gatttttgtg atgctcgtca ggggggccga 2340
   gcctatggaa aaacgccagc aacgcggcct ttttacggtt cctggccttt tgctggcctt 2400
   ttgctcacat gttctttcct gcgttatccc ctgattctgt ggataaccgt attaccgcct 2460
   ttgagtgagc tgataccgct cgccgcagcc gaacgaccga gcgcagcgag tcagtgagcg 2520
   aggaagcgga agagcgccca atacgcaaac cgcctctccc cgcgcgttgg ccgattcatt 2580
   aatgcagctg gcacgacagg tttcccgact ggaaagcggg cagtgagcgc aacgcaatta 2640
   atgtgagtta cctcactcat taggcacccc aggctttaca ctttatgctt ccggctccta 2700
   tgttgtgtgg aattgtgagc ggataacaat ttcacacagg aaacagctat gaccatgatt 2760
   acgccaagct cggaattaac cctcactaaa gggaacaaaa gctggtaccg atcccgagct 2820
   ttgcaaatta aagccttcga gcgtcccaaa accttctcaa gcaaggtttt cagtataatg 2880
   ttacatgcgt acacgcgtct gtacagaaaa aaaagaaaaa tttgaaatat aaataacgtt 2940
   cttaatacta acataactat aaaaaaataa atagggacct agacttcagg ttgtctaact 3000
   ccttcctttt cggttagagc ggatgtgggg ggagggcgtg aatgtaagcg tgacataact 3060
   aattacatga tatcgacaaa ggaaaagggg cctgtttact cacaggcttt tttcaagtag 3120
   gtaattaagt cgtttctgtc tttttccttc ttcaacccac caaaggccat cttggtactt 3180
   tttttttttt tttttttttt tttttttttt tttttttttt tttttttttt tttttttttt 3240
   tttttttttt tttttttttt tttttttttt ttcatagaaa taatacagaa gtagatgttg 3300
   aattagatta aactgaagat atataattta ttggaaaata catagagctt tttgttgatg 3360
   cgcttaagcg atcaattcaa caacaccacc agcagctctg attttttctt cagccaactt 3420
   ggagacgaat ctagctttga cgataactgg aacatttgga attctaccct tacccaagat 3480
   cttaccgtaa ccggctgcca aagtgtcaat aactggagca gtttccttag aagcagattt 3540
   caagtattgg tctctcttgt cttctgggat caatgtccac aatttgtcca agttcaagac 3600
   tggcttccag aaatgagctt gttgcttgtg gaagtatctc ataccaacct taccgaaata 3660
   acctggatgg tatttatcca tgttaattct gtggtgatgt tgaccaccgg ccatacctct 3720
   accaccgggg tgctttctgt gcttaccgat acgaccttta ccggctgaga cgtgacctct 3780
   gtgctttcta gtcttagtga atctggaagg cattcttgat tagttggatg attgttctgg 3840
   gatttaatgc aaaaatcact taagaaggaa aatcaacgga gaaagcaaac gccatcttaa 3900
   atatacggga tacagatgaa agggtttgaa cctatctgga aaatagcatt aaacaagcga 3960
   aaaactgcga ggaaaattgt ttgcgtctct gcgggctatt cacgcgccag aggaaaatag 4020
   gaaaaataac agggcattag aaaaataatt ttgattttgg taatgtgtgg gtcctggtgt 4080
   acagatgtta cattggttac agtactcttg tttttgctgt gtttttcgat gaatctccaa 4140
   aatggttgtt agcacatgga agagtcaccg atgctaagtt atctctatgt aagctacgtg 4200
   gcgtgacttt tgatgaagcc gcacaagaga tacaggattg gcaactgcaa atagaatctg 4260
   gggatccccc ctcgacggat gcaagggttc gaatccctta gctctcatta ttttttgctt 4320
   tttctcttga ggtsgtcaca tgatcgcaaa atggcaaatg gcacgtgaag ctgtcgatat 4380
   tggggaactg tggtggttgg caaatgacta attaagttag tcaaggcgcc atcctcatga 4440
   aaactgtgta acataataac cgaagtgtcg aaaaggtggc accttgtcca attgaacacg 4500
   ctcgatgaaa aaaataagat atatataagg ttaagtaaag cgtctgttag aaaggaagtt 4560
   tttccttttt cttgctctct tgtcttttca tctactattt ccttcgtgta atacagggtc 4620
   gtcagataca tagatacaat tctattaccc ccatccatac atctagaact agtggatccc 4680
   ccgggctgca ggaattcgat atcaagcttc acagctagcg cactcggtgc cccgcgcagg 4740
   gtcgcgatgc tgcccggttt ggcactgttc ctgctggccg cctggacggc tcgggcgctg 4800
   gatgcagaat tccgacatga ctcaggatat gaagttcatc atcaaaaatt ggtgttcttt 4860
   gcagaagatg tgggttcaaa caaaggtgca atcattggac tcatggtggg cggtgttgtc 4920
   atagcgacag tgatcgtcat caccttggtg atgctgaaga agaaacagta cacatccatt 4980
   catcatggtg tggtggaggt tgacgccgct gtcaccccag aggagcgcca cctgtccaag 5040
   atgcagcaga acggctacga aaatccaacc tacaagttct ttgagcagat gcagaacgcg 5100
   cggggtaccc cggcgatgaa gctactgtct tctatcgaac aagcatgcga tatttgccga 5160
   cttaaaaagc tcaagtgctc caaagaaaaa ccgaagtgcg ccaagtgtct gaagaacaac 5220
   tgggagtgtc gctactctcc caaaaccaaa aggtctccgc tgactagggc acatctgaca 5280
   gaagtggaat caaggctaga aagactggaa cagctatttc tactgatttt tcctcgagaa 5340
   gaccttgaca tgattttgaa aatggattct ttacaggata taaaagcatt gttaacagga 5400
   ttatttgtac aagataatgt gaataaagat gccgtcacag atagattggc ttcagtggag 5460
   actgatatgc ctctaacatt gagacagcat agaataagtg cgacatcatc atcggaagag 5520
   agtagtaaca aaggtcaaag acagttgact gtatcgccgg aattcccggg gatctgggcc 5580
   cccccgaccg atgtcagcct gggggacgag ctccacttag acggcgagga cgtggcgatg 5640
   gcgcatgccg acgcgctaga cgatttcgat ctggacatgt tgggggacgg ggattccccg 5700
   gggccgggat ttacccccca cgactccgcc ccctacggcg ctctggatat ggccgacttc 5760
   gagtttgagc agatgtttac cgatgccctt ggaattgacg agtacggtgg gtagggatcc 5820
   actagtccag tgtggtggaa ttctgcagat atccagcaca gtggcggccg ctcgaccccg 5880
   ggtgctagca aggccttgtg gccagccatg gcaactagtg cggccgctaa gtaagtaaga 5940
   cgtcgagctc taagtaagta acggccgcca ccgcggtgga gctttggact tcttcgccag 6000
   aggtttggtc aagtctccaa tcaaggttgt cggcttgtct accttgccag aaatttacga 6060
   aaagatggaa aagggtcaaa tcgttggtag atacgttgtt gacacttcta aataagcgaa 6120
   tttcttatga tttatgattt ttattattaa ataagttata aaaaaaataa gtgtatacaa 6180
   attttaaagt gactcttagg ttttaaaacg aaaattcttg ttcttgagta actctttcct 6240
   gtaggtcagg ttgctttctc aggtatagca tgaggtcgct cttattgacc acacctctac 6300
   cggcatgccg agcaaatgcc tgcaaatcgc tccccatttc acccaattgt agatatgcta 6360
   actccagcaa tgagttgatg aatctcggtg tgtattttat gtcctcagag gacaatacct 6420
   gttgtaatcg ttcttccaca cggatcccaa ttcgccctat agtgagtcgt attacaattc 6480
   actggccgtc gttttacaac gtcgtgactg ggaaaaccct ggcgttaccc aacttaatcg 6540
   ccttgcagca catccccctt tcgccagctg gcgtaatagc gaagaggccc gcaccgatcg 6600
   cccttcccaa cagttgcgca gcctgaatgg cgaatggacg cgccctgtag cggcgcatta 6660
   agcgcggcgg gtgtggtggt tacgcgcagc gtgaccgcta cacttgccag cgccctagcg 6720
   cccgctcctt tcgctttctt cccttccttt ctcgccacgt tcgccggctt tccccgtcaa 6780
   gctctaaatc gggggctccc tttagggttc cgatttagtg ctttacggca cctcgacccc 6840
   aaaaaacttg attagggtga tggttcacgt agtgggccat cgccctgata gacggttttt 6900
   cgccctttga cgttggagtc cacgttcttt aatagtggac tcttgttcca aactggaaca 6960
   acactcaacc ctatctcggt ctattctttt gatttataag ggattttgcc gatttcggcc 7020
   tattggttaa aaaatgagct gatttaacaa aaatttaacg cgaattttaa caaaatatta 7080
   acgtttacaa tttcctgatg cggtattttc tccttacgca tctgtgcggt atttcacacc 7140
   gcaggcaagt gcacaaacaa tacttaaata aatactactc agtaataacc tatttcttag 7200
   catttttgac gaaatttgct attttgttag agtcttttac accatttgtc tccacacctc 7260
   cgcttacatc aacaccaata acgccattta atctaagcgc atcaccaaca ttttctggcg 7320
   tcagtccacc agctaacata aaatgtaagc tttcggggct ctcttgcctt ccaacccagt 7380
   cagaaatcga gttccaatcc aaaagttcac ctgtcccacc tgcttctgaa tcaaacaagg 7440
   gaataaacga atgaggtttc tgtgaagctg cactgagtag tatgttgcag tcttttggaa 7500
   atacgagtct tttaataact ggcaaaccga ggaactcttg gtattcttgc cacgactcat 7560
   ctccatgcag ttggacgata tcaatgccgt aatcattgac cagagccaaa acatcctcct 7620
   taggttgatt acgaaacacg ccaaccaagt atttcggagt gcctgaacta tttttatatg 7680
   cttttacaag acttgaaatt ttccttgcaa taaccgggtc aattgttctc tttctattgg 7740
   gcacacatat aatacccagc aagtcagcat cggaatctag agcacattct gcggcctctg 7800
   tgctctgcaa gccgcaaact ttcaccaatg gaccagaact acctgtgaaa ttaataacag 7860
   acatactcca agctgccttt gtgtgcttaa tcacgtatac tcacgtgctc aatagtcacc 7920
   aatgccctcc ctcttggccc tctccttttc ttttttcgac cgaattaatt cttaatcggc 7980
   aaaaaaagaa aagctccgga tcaagattgt acgtaaggtg acaagctatt tttcaataaa 8040
   gaatatcttc cactactgcc atctggcgtc ataactgcaa agtacacata tattacgatg 8100
   ctgtctatta aatgcttcct atattatata tatagtaatg tcgtttatgg tgcactctca 8160
   gtacaatctg ctctgatgcc gcatagttaa gccagccccg acacccgcca acacccgctg 8220
   acgcgccctg acgggcttgt ctgctcccgg catccgctta cagacaagct gtgaccgtct 8280
   ccgggagctg catgtgtcag aggttttcac cgtcatcacc gaaacgcgcg a 8331
<210> 13
   <211> 355
   <212> PRT
   <213> Künstliche Sequenz
<220>
   <223> Beschreibung der künstlichen Sequenz: Mutagen
<400> 13
   Met Leu Pro Gly Leu Ala Leu Phe Leu Leu Ala Ala Trp Thr Ala Arg
   1 5 10 15
   Ala Leu Asp Ala Glu Phe Arg His Asp Ser Gly Tyr Glu Val His His
   20 25 30
   Gln Lys Leu Val Phe Phe Ala Glu Asp Val Gly Ser Asn Lys Gly Ala
   35 40 45
   Ile Ile Gly Leu Met Val Gly Gly Val Val Ile Ala Thr Val Ile Val
   50 55 60
   Ile Thr Leu Val Met Leu Lys Lys Lys Gln Tyr Thr Ser Ile His His
    65                 70                  75                  80
   Gly Val Val Glu Val Asp Ala Ala Val Thr Pro Glu Glu Arg His Leu
                   85                  90                  95
   Ser Lys Met Gln Gln Asn Gly Tyr Glu Asn Pro Thr Tyr Lys Phe Phe
               100                 105                 110
   Glu Gln Met Gln Asn Ala Arg Gly Thr Pro Ala Met Lys Leu Leu Ser
           115                 120                 125
   Ser Ile Glu Gln Ala Cys Asp Ile Cys Arg Leu Lys Lys Leu Lys Cys
       130                 135                 140
   Ser Lys Glu Lys Pro Lys Cys Ala Lys Cys Leu Lys Asn Asn Trp Glu
   145                 150                 155                 160
   Cys Arg Tyr Ser Pro Lys Thr Lys Arg Ser Pro Leu Thr Arg Ala His
                   165                 170                 175
   Leu Thr Glu Val Glu Ser Arg Leu Glu Arg Leu Glu Gln Leu Phe Leu
               180                 185                 190
   Leu Ile Phe Pro Arg Glu Asp Leu Asp Met Ile Leu Lys Met Asp Ser
           195                 200                 205
   Leu Gln Asp Ile Lys Ala Leu Leu Thr Gly Leu Phe Val Gln Asp Asn
       210                 215                 220
   Val Asn Lys Asp Ala Val Thr Asp Arg Leu Ala Ser Val Glu Thr Asp
   225                 230                 235                 240
   Met Pro Leu Thr Leu Arg Gln His Arg Ile Ser Ala Thr Ser Ser Ser
                   245                 250                 255
   Glu Glu Ser Ser Asn Lys Gly Gln Arg Gln Leu Thr Val Ser Pro Glu
               260                 265                 270
   Phe Pro Gly Ile Trp Ala Pro Pro Thr Asp Val Ser Leu Gly Asp Glu
           275                 280                 285
   Leu His Leu Asp Gly Glu Asp Val Ala Met Ala His Ala Asp Ala Leu
       290                 295                 300
   Asp Asp Phe Asp Leu Asp Met Leu Gly Asp Gly Asp Ser Pro Gly Pro
   305                 310                 315                 320
   Gly Phe Thr Pro His Asp Ser Ala Pro Tyr Gly Ala Leu Asp Met Ala
                   325                 330                 335
   Asp Phe Glu Phe Glu Gln Met Phe Thr Asp Ala Leu Gly Ile Asp Glu
               340                 345                 350
   Tyr Gly Gly
           355

## Beschreibung

Die Erfindung betrifft ein Verfahren zur Bestimmung der γ-Sekretase Aktivität, einzelne Komponenten des Verfahrens und die Anwendung des Verfahrens.

Bei der Alzheimer'schen Krankheit (Morbus Alzheimer) handelt es sich um eine neurodegenerative Erkrankung des Gehirns, die auf zellulärer Ebene mit einem massiven Verlust von Neuronen im limbischen System und im cerebralen Cortex einher geht. Auf molekularer Ebene lassen sich in den betroffenen Gehirnarealen Proteinablagerungen, sogenannte Plaques, nachweisen, die ein wesentliches Charakteristikum der Alzheimer'schen Krankheit darstellen. Das am häufigsten in diesen Plaques vorkommende Protein ist ein 40 bis 42 Aminosäuren großes Peptid, das als Aß-Peptid bezeichnet wird. Bei diesem Peptid handelt es sich um ein Spaltprodukt eines wesentlich größeren Proteins von 695 bis 770 Aminosäuren, dem sogenannten Amyloidvorläuferprotein (Amyloid Precursor Protein; APP).

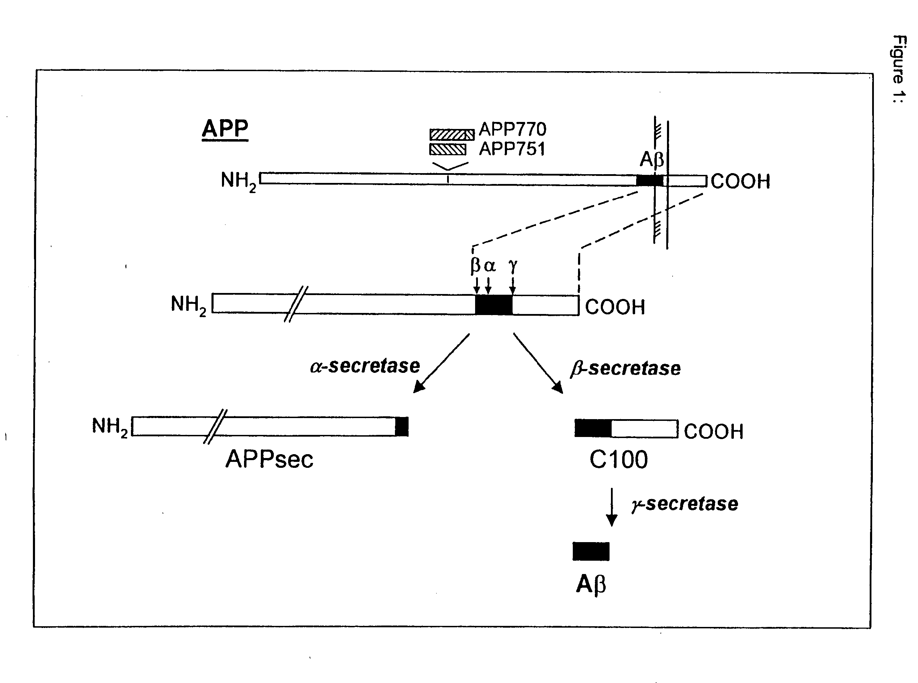

APP ist ein integrales Transmembranprotein, das die Lipiddoppelschicht einmal durchquert. Der weitaus größte Teil des Proteins liegt extrazellulär, während die kürzere C-terminale Domäne in das Cytosol gerichtet ist (Figur 1). Das Aß-Peptid ist in Figur 1 dunkelgrau dargestellt. Etwa zwei Drittel des Aß-Peptids stammen aus der extrazellulären Domäne und etwa ein Drittel aus der Transmembrandomäne von APP.

Neben dem membranständigen APP läßt sich eine sekretierte Form des Amyloid Vorläuferproteins nachweisen, die aus der großen Ektodomäne des APPs besteht und als APPsec ("sekretiertes APP") bezeichnet wird. APPsec entsteht aus APP durch proteolytische Spaltung, die durch die α-Sekretase erfolgt. Die proteolytische Spaltung findet an einer Stelle der Aminosäuresequenz von APP statt, die innerhalb der Aminosäuresequenz des Aß-Peptids liegt (nach Aminosäurerest 16 des Aß-Peptids). Eine Proteolyse von APP durch die α-Sekretase schließt damit die Bildung des Aß-Peptids aus.

Das Aß-Peptid kann also nur auf einem alternativen Prozessierungsweg aus APP gebildet werden. Es wird postuliert, daß an diesem Prozessierungsweg zwei weitere Proteasen beteiligt sind, wobei die eine Protease, die als β-Sekretase bezeichnet wird, am N-Terminus des Aß-Peptids im APP schneidet und die zweite Protease, die als γ-Sekretase bezeichnet wird, den C-Terminus des Aß-Peptids freisetzt (Kang, J. et al., Nature, 325, 733) (Figur 1).

Bisher konnte keine der drei Sekretasen bzw. Proteasen (α-Sekretase, β-Sekretase, γ-Sekretase) identifiziert werden. Die Kenntnis der Sekretasen ist jedoch von großem Interesse, insbesondere im Rahmen von Untersuchungen zur Alzheimer'schen Krankheit und zur Identifizierung der beteiligten Proteine, die dann wiederum als Targets in weiterführenden Studien eingesetzt werden können. Zum einen könnte die Inhibition der β- und insbesondere der γ-Sekretase zu einer Reduktion der Aß-Produktion führen, zum anderen könnte eine Aktivierung der α-Sekretase die Prozessierung von APP in APPsec steigern und würde damit gleichzeitig die Entstehung des Aß-Peptids reduzieren. Ein transgener C. elegans, der im Rahmen solcher Untersuchungen Anwendung finden wird, ist in der unveröffentlichten Deutschen Patentanmeldung mit dem Aktenzeichen 198 49 073.9 beschrieben.

Es gibt viele Hinweise darauf, daß das Aß-Peptid bei dem Auftreten der Alzheimer'schen Krankheit ein entscheidender Faktor ist. Unter anderem wird eine Neurotoxizität von Aß-Fibrillen in Zellkultur postuliert (Yankner, B.A. et al., (1990) Proc Natl Acad Sci USA,87, 9020). Auch tritt bei Patienten mit Down Syndrom, bei denen APP in einer zusätzlichen Kopie vorkommt die für den Morbus Alzheimer charakteristische Neuropathologie bereits im Alter von 30 Jahren auf. Dabei wird angenommen, daß der Überexpression von APP eine erhöhte Umsetzung in das Aß-Peptid folgt (Rumble, B. et al., (1989), N. Engl. J. Med., 320, 1446).
Den vielleicht stärksten Hinweis auf die zentrale Rolle des Aß-Peptids stellen die familiären Formen der Alzheimer'schen Krankheit dar. Hier finden sich Mutationen im APP-Gen um den Bereich der β- und γ-Sekretaseschnittstellen oder in zwei weiteren AD-assoziierten Genen (Presenilinen), die in Zellkultur zu einer wesentlichen Erhöhung der Aß-Produktion führen (Scheuner, D. et al., (1996), Nature Medicine, 2, 864).

Es gibt eine Reihe von Hinweisen darauf, daß APP bei seiner Prozessierung in das Aß-Peptid zunächst durch die β-Sekretase geschnitten wird um im Anschluß daran als Substrat für die γ-Sekretase zu dienen (Maruyama, K. Y. et al., (1994) Biochem. Biophys Res Commun, 202, 1517; Estus, S. et al., (1992), Science, 255, 726). Der γ-Sekretase kommt daher bei der Entstehung des Aß-Peptids eine entscheidende Rolle zu. Als Nachweis für die Aktivität der γ-Sekretase dient üblicherweise die Detektion des Aß-Peptids die sich allerdings häufig schwierig gestaltet.

Wolfe et al., Journal of Medicinal Chemistry, Bd. 41, 1998, Seiten 6 - 9, beschreibt die γ-Sekretase als das Enzym, welches in der Transmembranregion von APP proteolytisch schneidet, und letztlich das Aß-Peptid freisetzt. Die proteolytische Schnittstelle der γ-Sekretase wird in Figur 1 dargestellt und ist bekannt. Die Bedeutung der γ-Sekretase Aktivität für die Entstehung der Alzheimerschen Krankheit und als Angriffspunkt bei der Entwicklung therapeutischer Wirkstoffe wird erkannt und Inhibitoren für das Enzym werden vorgestellt. Mit Hilfe von SDS-PAGEs wird die Generierung des Aβ-Peptids beobachtet, dessen Entstehung und Menge in eigens transfizierten Zellen gemessen wird und als Parameter für die Aktivität der γ-Sekretase angesehen wird, siehe hierzu die Figuren 2 und 3.

Ein wesentlicher Grund hierfür liegt darin, daß nur ein geringer Teil von APP in das Aß-Peptid umgesetzt wird (Simons M, et al., Neurosci (1996) 1;16(3):899-908). Darüber hinaus ist das Aß-Peptid ein nur sehr kleines Bruchfragment von etwa 4 kDa und besitzt aufgrund seines hydrophoben Charakters eine starke Tendenz zur Selbstaggregation so daß es unter physiologischen Bedingungen leicht ausfällt (Hilbich, C. et al., (1991) J. Mol. Biol., 218, 149).

Der Nachweis des Aß-Peptids in eukaryotischen Zellen erfolgt über immunobiologische Methoden wie z.B. ELISA, Immunpräzipitation und Western-. Blotting (Suzuki, N. et al., Science 1994, 27, 264(5163) 1336; Haass, C. et al., (1992) Nature, 359, 322). Diese Verfahren sind relativ aufwendig, da sie die Inkubation mit entsprechenden Antikörpern implizieren und einen Aufschluß der verwendeten Zellen aus Zellkultur oder Modellorganismen (u.a. *C. elegans*) erfordern.

Gegenstand der vorliegenden Erfindung ist ein neues Verfahren zur Bestimmung der γ-Sekretase Aktivität und zum Nachweis von γ-Sekretase; besondere Ausführungsformen des Verfahrens betreffen einerseits Verfahren zur Identifizierung einer γ-Sekretase bzw. einer cDNA, die für eine γ-Sekretase kodiert und andererseits Verfahren zur Identifizierung von Substanzen, die die Aktivität einer γ-Sekretase inhibieren können. Solchen Substanzen kommt eine besondere Bedeutung zu, da sie z.B. als pharmazeutische Wirkstoffe, z.B. zur Behandlung der Alzheimer'schen Krankheit, verwendet werden können.

Gegenstand der Erfindung ist ein Verfahren zum Nachweise der Aktivität von γ-Sekretase, wobei
1. ein Transgen bereitgestellt bzw. verwendet wird, das ein Fusionsprotein kodiert und folgende Bestandteile enthält:
   a) eine erste Nukleotidsequenz, die für ein Protein, das die Aminosäuresequenz GAIIGLMVGGWIATVIVITLVML (SEQ ID NO. 1) enthält, kodiert,
   b) am 5'-Ende der ersten Nukleotidsequenz eine zweite Nukleotidsequenz, die für ein Signalpeptid kodiert,
   c) einen Promotor und
   d) gegebenenfalls weitere kodierende und/oder nicht-kodierende Nukleotidsequenzen;
2. dieses Transgen in eine Zelle eingebracht und das Fusionsprotein exprimiert wird;
3. das Fusionsprotein durch in der Zelle vorliegende γ-Sekretase innerhalb der Aminosäuresequenz SEQ ID NO. 1 gespalten wird, wodurch ein erstes Teilprotein, das die Aminosäuresequenz GAIIGLMVGGVV (SEQ ID NO. 2) enthält und ein zweites Teilprotein, das die Aminosäuresequenz VIVITLVML (SEQ ID NO. 3) enthält, gebildet werden;
4. die Menge an zweitem Teilprotein bestimmt und aus der Menge an gebildetem zweitem Teilprotein die Aktivität der γ-Sekretase bestimmt wird.

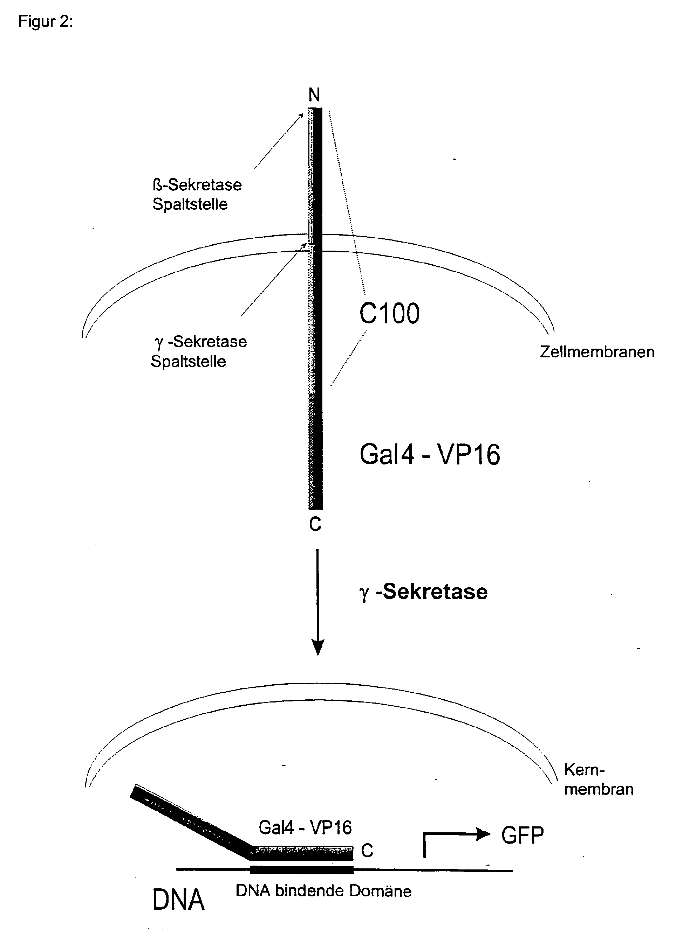

Die Verfahren ("Aß-Peptid Screening Assay", "γ-Sekretase Assay") sind zum *in vivo* Nachweis einer γ-Sekretase bzw. der Aktivität einer γ-Sekretase geeignet, wobei die Verfahren universell, auch z.B. im Hochdurchsatzscreening ("HTS") eingesetzt werden können. Die Verfahren weisen die oben erwähnten Nachteile herkömmlicher Nachweisverfahren nicht auf, insbesondere sind aufwendige Isolierungs- und Detektionsschritte nicht notwendig. Grundlage der Verfahren ist, daß das C-terminale APP-Fragment, das durch die γ-Sekretase in zwei Fragmente - ein erstes Teilprotein, das die Aminosäuresequenz GAIIGLMVGGVV (SEQ ID NO. 2) enthält und ein zweites Teilprotein, das die Aminosäuresequenz VIVITLVML (SEQ ID NO. 3) enthält - geschnitten wird, wobei das zweite Teilprotein, das die Aminosäuresequenz VIVITLVML (SEQ ID NO. 3) enthält, in das Cytosol der Zelle diffundiert (Figur 2). Im Cytosol einer Zelle kann dieses zweite Teilprotein, z.B. mit Hilfe eines Reportergens, leicht detektiert werden; es dient als Nachweis für eine γ-Sekretase bzw. die Aktivität einer γ-Sekretase. Die γ-Sekretaseschnittstelle ist in der Transmembrandomäne des APP lokalisiert (Kang, J. et al., (1987) Nature, 325, 733). Die APP-Transmembrandomäne hat die Aminosäuresequenz GAIIGLMVGGVV₄₀ IA₄₂ TVIVITLVML. Die γ-Sekretase schneidet nach V₄₀, A₄₂ oder T₄₃. Im Gegensatz dazu wird das Aß-Peptid, das von eukaryotischen Zelle in Zellkultur produziert wird, in den Mediumüberstand sekretiert.

Mit Hilfe eines geeigneten Reportersystems kann die Freisetzung des zweiten Teilproteins die Expression eines Reporterproteins aktivieren, das in eukaryotischen Zellen detektiert werden kann. Durch den Nachweis des Reporterproteins kann nachgewiesen werden, daß ein γ-Sekretaseschnitt im APP stattgefunden hat. Dadurch kann die γ-Sekretase bzw. die Aktivität der γ-Sekretase qualitativ und/oder quantitativ bestimmt werden.

Die Bestandteile des Verfahrens können wie folgt näher charakterisiert werden:

Die erste Nukleotidsequenz kodiert für ein Amyloid Vorläuferprotein (APP) oder einen Teil davon. Vorzugsweise kodiert die erste Nukleotidsequenz für ein Protein, das die Aminosäuresequenz (SEQ ID NO. 4) enthält; SEQ ID NO. 4 enthält SEQ ID NO. 1.

Die zweite Nukleotidsequenz kodiert für ein Signalpeptid, vorzugsweise für das Signalpeptid von APP (nachfolgend "SP" abgekürzt). Das Signalpeptid enthält beispielsweise die Aminosäuresequenz SEQ ID NO. 5.

Als Promotor kann ein regulierbarer oder ein konstitutiver Promotor verwendet werden. Der Promotor kann beispielsweise für die Expression in Säugetierzellen, in C. elegans, in Hefe oder in Drosophila geeignet sein. Als Promotoren für Säugetierzellen sind z.B. der CMV (z.B: Clontech, Heidelberg, Deutschland), HSV TK (z.B. Clontech), RSV (z.B. Invitrogen, NV Leek, Niederlande), SV40 (z.B. Clontech) und LTR (z.B. Clontech) geeignet. Als Promotoren für C. elegans können z.B. unc119, unc54, hsp16-2, G₀A1 und sel-12 verwendet werden. Für die Expression in Hefe sind die Promotoren ADH1 (konstitutiv) (Vickova et al. (1994) Gene, 25(5), 472-4), Gal1 (konditionell induzierbar) (Selleck et al. (1987) Nature 325, 173-7), MET3 (konditionell) (Cherest et al. (1987) Mol Gen Genet 210, 307-13) und Met 25 geeignet. In Drosophila können z.B. die Promotoren MT (Metallothionin) (z.B. lnvitrogen), Ac5 (Invitrogen) oder Ds47 (Invitrogen) verwendet werden.

Vorzugsweise wird in dem Verfahren eine eukaryotische Zelle eingesetzt, beispielsweise eine humane Zelle oder eine nicht-humane Zelle, z.B. Affe, Hamster, Maus, Drosophila Zebrafisch oder Hefe. Beispielsweise kann eine HeLa, 293, H4, SH-SY5Y, H9, Cos, CHO, N2A, SL-2 oder Saccharomyces cerevisiae Zelle eingesetzt werden. In einer besonderen Ausführungsform der Erfindung wird eine C. elegans Zelle eingesetzt. Die Zelle kann Bestandteil eines transgenen, nicht-humanen Tieres sein. In einer besonderen Ausführungsform kann die transgene Zelle Bestandteil eines transgenen C. elegans sein. Insbesondere betrifft die Erfindung Verfahren, bei denen Hefezellen, z.B. vom Stamm MAV203 (Life Technologies, Rockville, MD, USA) oder EGY 48 (OriGene Technologies, Inc. Rockville, MD, USA), verwendet werden.

Das Transgen kodiert für ein Fusionsprotein; dies setzt sich zusammen aus den Teilproteinen die durch die erste und die zweite Nukleotidsequenz und gegebenenfalls weitere Nukleotidsequenzen kodiert werden. Das Fusionsprotein enthält also das erste Teilprotein und das zweite Teilprotein und ggf. ein weiteres Teilprotein. Das Fusionsprotein hat beispielsweise die Aminosäuresequenz SEQ ID NO. 6.

Insbesondere kann in dem Verfahren ein Transgen eingesetzt werden, das die Nukleotidsequenz SEQ ID NO. 8 hat. In besonderes bevorzugten Ausführungsformen des Verfahrens liegt das Transgen in einem Vektor vor. Der rekombinante Vektor kann die Nukleotidsequenz SEQ ID NO. 9 haben. Diese spezielle Ausführungsform der Erfindung wird auch als SP-C100-Gal 4-VP16 System bezeichnet. Dabei wird ein Fusionsprotein bestehend aus dem Signalpeptid von APP, dem C100 Fragment von APP, GaI4 und VP16 exprimiert. Dieses in der Transmembrandomäne lokalisierte Protein wird innerhalb des C100 Fragments gespalten und das zweite Teilprotein, d.h. der Teil des Fusionsproteins, der einen Teil des C100 Fragments, Gal4 und VP16 enthält wird mit Hilfe eines Reporterplasmids nachgewiesen.

Neben dem Transgen Konstrukt SPC100-Gal4-VP16 sind auch andere Reporterkonstrukte denkbar, bei denen z.B. die transkriptionsaktivierende Domäne zwischen die Transmembrandomäne und cytosolische Domäne von SPC100 oder ein Tag (z.B. MYC, FLAG) an den N- und C-Terminus und zwischen die Transmembran- und die cytosolische Domäne von SPC100 eingefügt sein könnte.

Die weitere kodierende Nukleotidsequenz kann beispielsweise für ein Protein kodieren, das zur Detektion des zweiten Teilproteins verwendet werden kann. Vorzugsweise ist die weitere kodierende Nukleotidsequenz deshalb am 3'-Ende der ersten Nukleotidsequenz lokalisiert. Die weitere kodierende Nukleotidsequenz kodiert beispielsweise für ein chimeres Protein oder ein anderes Protein, das aus mehreren Domänen aufgebaut ist, z.B. ein Protein das eine DNA-bindende Domäne und eine transkriptionsaktivierende Domäne enthält. In einer besonderen Ausführungsform der Erfindung kodiert die weitere kodierende Nukleotidsequenz für ein Protein, das aus einer Ga14-bindenden Domäne und aus der Transkriptions-aktivierenden Domäne von VP16 besteht (GaI4-VP16), vorzugsweise hat das weitere Teilprotein dann die Aminosäuresequenz SEQ ID NO. 7. In Hefezellen kann das weitere Teilprotein auch eine LexA-bindende Domäne enthalten (z.B. Lex A-VP16). Dieses weitere Teilprotein ist insbesondere für Verfahren geeignet, in denen Zellen des Hefestammes EGY48 verwendet werden.

Insbesondere betrifft die Erfindung Verfahren, in denen Zellen verwendet werden, die mit einem Reporterplasmid co-transfiziert sind. Das Reporterplasmid enthält ein Reportergen unter der Kontrolle eines regulierbaren Promotors. Beispielsweise kann das Reportergen für GFP und dessen Derivate, z.B. EGFP (Enhanced Green Fluorescent Protein), EBFP, EYFP, d2EGFP, GFPuv oder Luciferase (z.B. Promega, Mannheim, Deutschland), CAT (z.B. P-romega), SEAP (z.B. Clontech), βGal (z.B. Clontech) oder Apoptose induzierende Faktoren, z.B. Fas, TNF-R1, death domain und Homologe (Tartaglia et al. (1993) Cell 74, 845-53), ced3, ced4, ced9 kodieren.

Das Reporterplasmid kann als regulierbarer Promotor z.B. einen Minimalpromotor von HIV, vom CD4-Promotor oder den mec7 Promotor enthalten. Die Wahl des geeigneten regulierbaren Promotor hängt von der verwendeten transkriptionsaktivierenden Domäne ab.

Eine besondere Ausführungsform der Erfindung betrifft die Durchführung des Verfahrens, wobei als Zellen Hefezellen verwendet werden. Als Alternative für den Hefe-Expressionsvektor pDBTRP (Life Technologies Inc.) (SEQ ID NO.: 11) in den in einer besonderen Ausführungsform der MET-25-Promotor eingesetzt wurde (siehe SEQ ID NO.: 12) können eine Vielzahl anderer Expressionsvektoren mit unterschiedlichen Promotoren (z.B. der induzierbare Gal1-Promotor und MET-25-Promotor oder der konstitutiv aktive ADH1-Promotor) und unterschiedlichen Selektionsmarkern (ADE, LEU, TRP, HIS, LYS, PHE) ausgewählt werden.

Eine besondere Ausführungsform betrifft die Verwendung von Hefezellen, die Gal4-oder LexA-induzierbare Reportergene stabil im Genom integriert oder extrachromosomal vorliegend enthalten.
Vorzugsweise werden für diese Ausführungsformen Hefen der Stämme MaV203 (Life Technologies, Rockville, MD, U.S.A.) oder EGY48 (Origene Technologies, Inc. Rockville, MD, U.S.A.) verwendet.

Eine besondere Ausführungsform der Verfahren betrifft die Verwendung einer Zelle, die zusätzlich mit einem weiteren rekombinanten Vektor transfiziert wurde. Vorzugsweise hat die Zelle, die für diese Ausführungsformen verwendet wird, normalerweise keine oder kaum endogene γ-Sekretase bzw. endogene γ-Sekretase Aktivtät ist mit den oben genannten Verfahren nicht nachweisbar. Diese Zelle kann mit einem weiteren Vektor transformiert worden, in dem eine Nukleotidsequenz - vorzugsweise eine cDNA - enthalten ist, die für eine γ-Sekretase kodiert. Beispielsweise kann eine cDNA Bank eingesetzt werden. Diese Ausführungsform des Verfahrens kann dann u.a. dazu verwendet werden, eine γ-Sekretase bzw. eine cDNA, die für eine γ-Sekretase kodiert, zu identifizieren. cDNA-Banken, die nach einer γ-Sekretase durchsucht werden können, können aus Zellen oder Geweben hergestellt werden, z.B. B-Zellen, Neuronen, Gliazellen, Hippocampus, Gesamtgehirn, Plazenta, Niere. Vorzugsweise wird die cDNA aus menschlichen Zellen bzw. menschlichen Geweben aber auch aus anderen Organismen (z.B. Hamster, Ratte, Maus, Hund, Affe) hergestellt.

Bei Zellen, die ohne Transfektion mit einer cDNA Bank keine, nach Transfektion mit einer cDNA Bank aber eine γ-Sekretase Aktivität zeigen, kann die in der Zelle vorliegende cDNA für eine γ-Sekretase kodieren. Aus Zellen die dieses Verhalten zeigen, kann diese cDNA nach bekannten Verfahren isoliert und verifiziert werden.

Vorzugsweise kodiert die erste Nukleotidsequenz für APP oder einen Teil von APP. Das Transgen kann beispielsweise die Nukleotidsequenz SEQ ID NO. 8 haben. Das Transgen kann in einem Vektor vorliegen. Dieser kann beispielsweise die Nukleotidsequenz SEQ ID NO. 9 haben.

Das Verfahren betrifft die Verwendung eines Transgens und/oder eines Vektors zur Herstellung einer transgenen Zelle, wobei die Zelle Bestandteil eines nicht-humanen Organismus sein kann. Beispielsweise, kann das Transgens und/oder der Vektor zur Herstellung eines transgenen C. elegans verwendet werden. In einer anderen besonderen Ausführungsform wird das Transgen und/oder der Vektor zur Herstellung von transgenen Hefezellen, z. B. S. cerevisiae Zellen verwendet.

Die Erfindung betrifft auch ein Verfahren zur Herstellung eines nicht-humanen Organismus, z.B. eines transgenen C. elegans, wobei ein Transgen und/oder ein Vektor, der ein Transgen enthält, in die Gonaden des Organismus, also z.B. eines C. elegans mikroinjiziert wird. Gegenstand der Erfindung ist auch eine Zelle, die ein erfindungsgemäßes Transgen enthält und ein transgener C. elegans, der ein erfindungsgemäßes Transgen enthält. Die Erfindung betrifft auch eine Zelle, insbesondere eine Hefezelle, die ein erfindungsgemäßes Transgen vorzugsweise vorliegend in einem geeigneten Vektor enthält. Gegenstand der Erfindung sind insbesondere Zellen, vorzugsweise Hefezellen, die das erfindungsgemäße Transgen und zusätzlich eine cDNA Bank enthalten.

Die Erfindung betrifft die Verwendung von transgenen bzw. rekombinanten Zellen, vorzugsweise Hefezellen oder eines transgenen C. elegans in einem Verfahren zur Bestimmung der γ-Sekretase bzw. der Aktivität der γ-Sekretase, die Verwendung dieser Zellen oder eines transgenen C. elegans in einem Verfahren zur Identifizierung von Inhibitoren der Aktivität der γ-Sekretase sowie das Verfahren selbst.

Insbesondere betrifft die Erfindung Verfahren zur Identifizierung von Substanzen, die die Aktivität einer γ-Sekretase inhibieren, wobei das Verfahren die folgenden Verfahrensschritte enthält:
1. Herstellung eines transgenen nicht-humanen Organismus, z.B. eines transgenen C. elegans oder Saccaromyces cerensiae oder einer transgenen Zelle, wobei der transgene nicht-humane Organismus oder die transgene Zelle ein Transgen enthält, das folgenden Bestandteile aufweist:
   a) eine erste Nukleotidsequenz, die für ein Protein, das die Aminosäuresequenz GAIIGLMVGGWIATVIVITLVML (SEQ ID NO. 1) enthält, kodiert,
   b) am 5'-Ende der ersten Nukleotidsequenz eine zweite Nukleotidsequenz, die für ein Signalpeptid kodiert und
   c) einen Promotor und der transgene nicht-humane Organismus oder die transgenen Zelle außerdem ein Reporterplasmid enthält, wobei das Reporterplasmid eine Proteinbindungsstelle, einen Minimalpromotor und ein Reportergen trägt und gegebenenfalls eine cDNA, die eine γ-Sekretase kodiert, wobei der transgene nicht-humane Organismus oder die transgene Zelle das Transgen und gegebenenfalls die durch die cDNA kodierte γ-Sekretase exprimiert;
2. der transgene nicht-humane Organismus oder die transgene Zelle mit einer zu untersuchenden Substanz inkubiert wird und
3. das Fusionsprotein durch in der Zelle vorliegende γ-Sekretase innerhalb der Aminosäuresequenz SEQ ID NO. 1 gespalten wird, wodurch ein erstes Teilprotein, das die Aminosäuresequenz GAIIGLMVGGW (SEQ ID NO. 2) enthält und ein zweites Teilprotein, das die Aminosäuresequenz VIVITLVML (SEQ ID NO. 3) enthält, gebildet werden und
4. die Menge an zweitem Teilprotein bestimmt und aus der Menge an gebildetem zweitem Teilprotein die Aktivität der γ-Sekretase bestimmt wird.

Die Erfindung betrifft auch ein Verfahren zur Identifizierung von Substanzen, die die Aktivität der γ-Sekretase inhibieren, wobei
1. ein Transgen bereitgestellt wird, das die folgenden Bestandteile enthält:
   a) eine erste Nukleotidsequenz, die für ein Protein, das die Aminosäuresequenz GAIIGLMVGGWIATVIVITLVML (SEQ ID NO. 1) enthält, kodiert,
   b) am 5'-Ende der ersten Nukleotidsequenz eine zweite Nukleotidsequenz, die für ein Signalpeptid kodiert und
   c) einen Promotor und
   d) gegebenenfalls weiter kodierende und/oder nicht-kodierende Nukleotidsequenzen;
2. dieses Transgen und ein Reporterplasmid und gegebenenfalls eine cDNA, die für eine γ-Sekretase kodiert, in eine Zelle eingebracht werden und das durch das Transgen kodierte Fusionsprotein und gegebenenfalls die durch die cDNA kodierte γ-Sekretase in Gegenwart einer zu untersuchenden Substanz exprimiert werden;
3. das Fusionsprotein durch in der Zelle vorliegende γ-Sekretase innerhalb der Aminosäuresequenz SEQ ID NO. 1
   a) gespalten oder
   b) nicht gespalten wird, wodurch entweder

   a) ein erstes Teilprotein, das die Aminosäuresequenz GAIIGLMVGGW (SEQ ID NO. 2) enthält und ein zweites Teilprotein, das die Aminosäuresequenz VIVITLVML (SEQ ID NO. 3) enthält, gebildet werden, oder
   b) keine nachweisbare Menge an ersten und/oder zweitem Teilprotein gebildet wird;
4. bestimmt wird, ob zweites Teilprotein gebildet wurde.

Unter anderem können die Verfahren z.B. in Verbindung mit dem C100-Gal 4-VP16 System (d.h. einem Fusionsprotein bestehend aus C100, Gal4 und VP16 bzw. unter Verwendung einer Nukleinsäure, die für ein entsprechendes Fusionsprotein kodiert) angewendet werden für:
1. Identifizierung und Bestimmung (qualitativ und/oder quantitativ) der Aktivität einer γ-Sekretase.
2. Identifizierung von γ-Sekretasen in unterschiedlichen Geweben, Zellen und Organismen bzw. Spezies. Identifizierung und Isolierung der betreffenden cDNA's, die für diese γ-Sekretase kodieren und die weitere Verwendung der cDNA's.
3. Screening in vivo, z.B. in Hefe-Zellen (Saccharomyces Cereviriae) oder C. elegans, wobei die Aktivität der γ-Sekretase ohne Verwendung immunbiologischer Methoden bestimmt werden kann.
4. Anwendung des Verfahrens zur Identifizierung und Charakterisierung von Substanzen, z.B. pharmakologischen Wirkstoffen, die die Aktivität der γ-Sekretase modulieren, z.B. Inhibitoren der γ-Sekretase. Insbesondere kann dieses Verfahren in einem HTS (Hochdurchsatz Screening) eingesetzt werden. Beispielsweise können Substanzen identifiziert werden, die zur Behandlung der Alzheimer'schen Krankheit und/ oder zur vorbeugenden Behandlung eingesetzt werden können.
5. Untersuchungen im Rahmen der Alzheimer'schen Krankheit, z.B. mit mutagenisiertem APP bzw. C100.
6. Die beschriebenen Fusionsproteine/Transgene, z.B. C100 in SP-C100-Gal 4-VP16 können durch Gesamt-APP ersetzt werden und mit Hilfe der Verfahren ebenfalls die γ-Sekretase, ihre Aktivität und Regulation untersucht werden.

Figur 1: Figur 1 zeigt das Amyloid Vorläuferprotein (Isoform APP695 und Isoformen APP770 bzw. APP751) und Sekretase Spaltprodukte.

Figur 2: Zeigt schematisch das den Verfahren zugrunde liegende Prinzip: β-Sekretase Spaltstelle am N-Terminus; γ-Sekretase Spaltstelle in der Transmembrandomäne; C100 = C100 Fragment von APP; Gal4-VP16 = DNA bindende Domäne, transkriptionsaktivierende Domäne (bestehend aus DNAbindender Domäne und Transkriptionsaktivator), die an die proteinbindende Domäne auf der DNA des Reporterplasmids bindet.

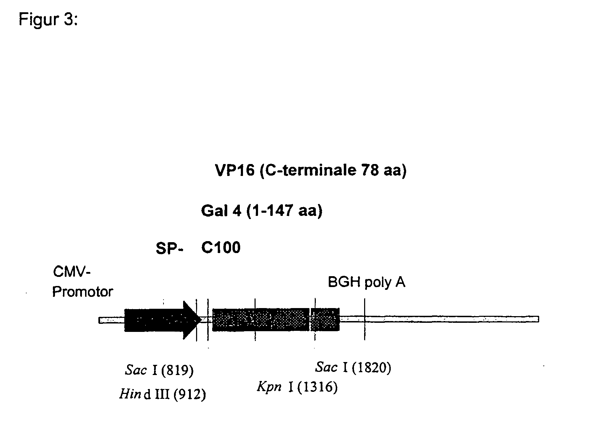

Figur 3: Konstruktion des Expressionsplasmids SP-C100-Gal4-VP16: aa= Aminosäuren; Restriktionsschnittstellen Sac I, Hind III und Kpn I unter Angabe der Position der Schnittstelle auf dem Plasmid.

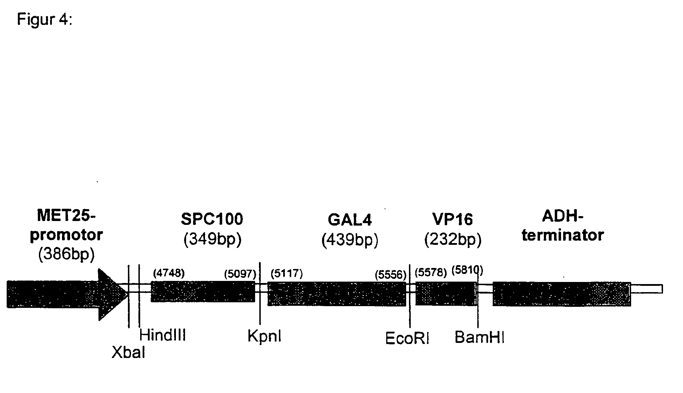

Figur 4: Expressionsplasmid pDBTRP-MET25-SP-C100-Gal4-VP16: Konstruktion des Expressionsplasmids für die Expression des Transgens in Hefe.

### Beispiele:

### Beispiel 1: Konstruktion des Expressionsplasmids SP-C100-Gal4-VP16

Das Plasmid kodiert das APP-Signalpeptid (SP), das an die C-terminalen 100 Aminosäurereste von APP (C100) fusioniert ist. C100 beginnt mit dem N-Terminus des Aß-Peptids und endet mit dem C-Terminus von APP. Es muß noch durch die γ-Sekretase geschnitten werden, um das Aß-Peptid freizusetzen.
An den C-Terminus von SP-C100 wurde Gal4-VP16 fusioniert. Gal4-VP16 setzt sich aus den ersten 147 Aminosäureresten des Hefe-Transkriptionsaktivators Gal4 und den 78 C-terminalen Aminosäureresten von VP16, eines Transkriptionsaktivators aus dem Herpes Simplex Virus zusammen. Als Fusionsprotein übernimmt das Gal 4 -Fragment die Funktion der DNA-Bindung während das VP16-Fragment die Transkription aktiviert (Sadowski et al., (1988) Science 335, 563). Als Vektorplasmid diente pcDNA3.1+ von der Firma Invitrogen, Niederlande.

### Beispiel 2: Konstruktion des Reporterplasmids pGL2 MRG5 EGFP

Das Reporterplasmid pGL2 MRG5 hat fünf Gal 4-Bindungsstellen vor der HIV-TATA-Box. Zur leichteren Detektion in Zellkultur wurde das Luciferasegen gegen das Gen für EGFP (Enhanced Green Fluorescent Protein) aus dem Vektor pEGFP N1 von der Firma Clontech, Heidelberg ausgetauscht.

### Beispiel 3:

Humane Neuroblastomzellen (SH-SYSY-Zellen) wurden mit beiden Plasmiden co-transfiziert und anschließend unter Bestrahlung mit Licht der Wellenlänge 480 nm, wodurch EGFP angeregt wird, mikroskopisch analysiert. Es konnten zum Teil sehr stark grün leuchtende Zellen detektiert werden.
Da dieser Effekt ebenfalls auf einer Expression des EGFP durch das Reporterplasmid ohne spezifische Aktivierung beruhen könnte, wurden SH-SY5Y Zellen nur mit Reporterplasmid transfiziert. Bei diesen Zellen war keine Grünfluoreszenz detektierbar. Die Expression muß daher durch Gal4-VP16 aktiviert werden, was eine proteolytische Freisetzung des APP-C-Terminus voraussetzt. Bislang sind abgesehen von der γ-Sekretase keine weiteren proteolytischen Aktivitäten beschrieben worden, die APP innerhalb der Transmembrandomäne oder im cytoplasmatischen Teil proteolytisch prozessieren. Daher wird angenommen, daß der Freisetzung des APP-C-Terminus, fusioniert an Gal 4-VP16, die Aktivität der γ-Sekretase zugrunde liegt.

### Beispiel 4:

Verwendung des C100-Gal4-VP16 Systems zur Detektion einer cDNA kodierend für eine γ-Sekretaseaktivität in cDNA-Banken:
SPC100-Gal4-VP16 wurde in den Hefe-Expressionsvektor pDBTRP (Life Technologies Rockville, MD, U.S.A.) unter Kontrolle des MET25-Promotors kloniert und der Hefestamm MaV203 (Life Technologies) mit diesen Konstrukten transformiert. Der Hefestamm MaV203 ist genetisch modifiziert und enthält drei GAL4 induzierbare Reporter Gene (URA3, HIS3, LacZ), die stabil in das Genom integriert sind. Die Expression der SPC1 00-Ga14-VPI 6 cDNA in MaV203 ergab eine nur geringe Aktivität der Reporter, so daß dieses System für eine Suche nach einer γ-Sekretase in einer cDNA Bank geeignet ist.

### Beispiel 5:

Die rekombinanten MaV203 Zellen aus Beispiel 4 können beispielsweise für die Identifizierung von γ-Sekretasen bzw. das Screenen einer humanen B-Zell cDNA Bank (American Type Culture Collection, Manassas, VA, U.S.A.) verwendet werden. Analog könnte auch eine humane hippocampale cDNA Bank, integriert in die Hefe-Expressionsvektoren p415-MET25 (ATCC, Nucleic Acid Research, 1994, Vol. 22, No. 25, 5767) oder p415-ADH1 (ATCC, GENE, 1995, 158: 119-122) für ein Screening nach einer cDNA, die für eine γ-Sekretase oder ein Protein mit γ-Sekretaseaktivität kodiert, eingesetzt werden.
(SEQ ID NO.: 1)
   GAIIGLMVGGWIATVIVITLVML
   Erstes Teilprotein (SEQ ID NO.: 2)
   GAIIGLMVGGVV
   Zweites Teilprotein (SEQ ID NO.: 3)
   VIVITLVML
   Aminosäuresequenz eines C100 -Fragments (SEQ ID NO.: 4) Aminosäuresequenz eines Signalpeptids von APP (SEQ ID NO.: 5)
   MLPGLALFLL AAWTARA
   Aminosäuresequenz eines SP-C100 Fragments (SEQ ID NO.: 6) Transkriptionsaktivierende Domäne VP16 mit GAL4 Bindungsdomäne (GAL4-VP16) (SEQ ID NO.: 7) Nukleotidsequenz eines Transgens kodierend für SP-C100-Ga14-VP16 (SEQ ID NO.: 8) Nukleotidsequenz von dem Säugetier-Expressionsvektor pcDNA3.1+ (Firma Invitrogen, Niederlande) (SEQ ID NO.: 9) Nukleotidsequenz von APP (SEQ ID NO.:10) Nukleotidsequenz des Plasmids pDBTRP (SEQ ID NO. 11): Nukleotidsequenz des rekombinanten Plasmids pDBTRP-MET25-SPC100-GAL4-VP16 (SEQ ID NO. 12): Aminosäuresequenz eines SP-C100-Ga14-VP16 Fusionsproteins (SEQ ID NO. 13):

### Literatur:

- Estus et al. (1992), Science, 255, 726.
   Haass et al. (1992) Nature, 359, 322.
- Hilbich et al. (1991) J. Mol. Biol., 218, 149
- Kang et al. (1987) Nature, 325, 733
- Maruyama et al. (1994) Biochem. Biophys Res Commun, 202, 1517
- Rumble et al. (1989), N. Engl. J. Med., 320, 1446
- Sadowski et al.(1988) Nature, 335, 563
- Scheuner et al. (1996), Nature Medicine, 2, 864
- Simons et al. Neurosci (1996) 1;16(3):899-908
- Suzuki et al. Science 1994 May 27;264(5163):1336-40
- Yankner et al. (1990) Proc Natl Acad Sci USA,87, 9020

## Patentansprüche

1. Verfahren zum Nachweise der Aktivität von γ-Sekretase, wobei
1. ein Transgen verwendet wird, das ein Fusionsprotein kodiert und folgende Bestandteile enhält:
a) eine erste Nukleotidsequenz, die für ein Protein, das die Aminosäuresequenz GAIIGLMVGGVVIATVIVITLVML (SEQ ID NO. 1) enthält, kodiert,
b) am 5'-Ende der ersten Nukleotidsequenz eine zweite Nukleotidsequenz, die für ein Signalpeptid kodiert,
c) einen Promotor und
d) gegebenenfalls weitere kodierende und/oder nicht-kodierende Nukleotidsequenzen;
2. dieses Transgen in eine Zelle eingebracht und das Fusionsprotein exprimiert wird;
3. das Fusionsprotein durch in der Zelle vorliegende γ-Sekretase innerhalb der Aminosäuresequenz SEQ ID NO. 1 gespalten wird, wodurch ein erstes Teilprotein, das die Aminosäuresequenz GAIIGLMVGGVV (SEQ CD NO. 2) enthält und ein zweites Teilprotein, das die Aminosäuresequenz VIVITLVML (SEQ ID NO. 3) enthält, gebildet werden und
4. die Menge an zweitem Teilprotein bestimmt und aus der Menge an gebildetem zweitem Teilprotein die Aktivität der γ-Sekretase bestimmt wird.

2. Verfahren nach einem der Ansprüche 1 und 2, wobei die erste Nukleotidsequenz für ein Amyloid Vorläufer Protein (APP) oder einen Teil davon kodiert.

3. Verfahren nach einem oder mehreren der Ansprüche 1 bis 3, wobei die erste Nukleotidsequenz für ein Protein kodiert, das die Aminosäuresequenz (SEQ ID NO. 4) enthält.

4. Verfahren nach einem oder mehreren der Ansprüche 1 bis 4, wobei die zweite Nukleotidsequenz für das Signalpeptid von APP (SP) kodiert.

5. Verfahren nach einem oder mehreren der Ansprüche 1 bis 5, wobei das Signalpeptid die Aminosäuresequenz SEQ ID NO. 5 enthält.

6. Verfahren nach einem oder mehreren der Ansprüche 1 bis 6, wobei der Promotor ein Promotor für die Expression in Säugetierzellen, in C. elegans, in Hefe oder in Drosophila ist:

7. Verfahren nach einem oder mehreren der Ansprüche 1 bis 7, wobei der Promotor der CMV, HSV TK, RSV, SV40, LTR, unc119, unc54, hsp16-2, GOA1, sel-12, ADH1, Gal1, MET3, MT, Ac5 oder Ds47 ist.

8. Verfahren nach einem oder mehreren der Ansprüche 1 bis 8, wobei die Zelle eine eukaryotische Zelle ist.

9. Verfahren nach einem oder mehreren der Ansprüche 1 bis 9, wobei die Zelle eine isolierte, nicht-embryonale humane Zelle ist.

10. Verfahren nach einem oder mehreren der Ansprüche 1 bis 10, wobei die Zelle eine nicht-humane Zelle ist.

11. Verfahren nach einem oder mehreren der Ansprüche 1 bis 11, wobei Zelle eine HeLa,293, H4, SH-SY5Y, H9, Cos, CHO, N2A, SL-2 oder Saccharomyces cerevisiae Zelle ist.

12. Verfahren nach einem oder mehreren der Ansprüche 1 bis 12, wobei die Zelle eine C. elegans Zelle ist.

13. Verfahren nach Anspruch 12, wobei die Zelle Bestandteil eines transgenen C. elegans ist.

14. Verfahren nach Anspruch 11, wobei die Zelle eine Hefezelle ist.

15. Verfahren nach einem oder mehreren der Ansprüche 1 bis 15, wobei das Fusionsprotein die Aminosäuresequenz SEQ ID NO. 6 hat oder enthält.

16. Verfahren nach einem oder mehreren der Ansprüche 1 bis 16, wobei die weitere kodierende Nukleotidsequenz am 3'-Ende der ersten Nukleotidsequenz lokalisiert ist.

17. Verfahren nach einem oder mehreren der Ansprüche 1 bis 17, wobei die weitere kodierende Nukleotidsequenz für ein Protein kodiert, das als Fusionsprotein mit dem ersten Teilprotein und dem zweiten Teilprotein exprimiert wird und zur Detektion des zweiten Teilproteins verwendet werden kann.

18. Verfahren nach einem oder mehreren der Ansprüche 1 bis 18, wobei die weitere kodierende Nukleotidsequenz für ein Protein kodiert, das eine DNA-bindende Domäne und eine transkriptionsaktivierende Domäne enthält.

19. Verfahren nach einem oder mehreren der Ansprüche 1 bis 19, wobei die weitere kodierende Nukleotidsequenz für ein Protein kodiert, das aus einer Gal4-bindenen Domäne und aus der Transkriptions-aktivierenden Domäne von VP 16 besteht (GaI4-VP16).

20. Verfahren nach einem oder mehreren der Ansprüche 1 bis 20, wobei die Zelle mit einem Reporterplasmid co-transfiziert wird, wobei das Reporterplasmid ein Reportergen unter der Kontrolle eines regulierbaren Promotors enthält.

21. Verfahren nach Anspruch 21, wobei der regulierbare Promotor durch die transkriptionsaktivierende Domäne aktivierbar ist.

22. Verfahren nach Anspruch 22, wobei das Reporterplasmid das Reportergen für EGFP (Enhanced Green Fluorescent Protein) kodiert und der regulierbare Promotor fünf Ga14-Bindungsstellen und einen Minimalpromotor von HIV enthält.

23. Verfahren nach einem oder mehreren der Ansprüche 1 bis 23, wobei das Transgen die Nukleotidsequenz SEQ ID NO. 8 hat.

24. Verfahren nach einem oder mehreren der Ansprüche 1 bis 24, wobei das Transgen in einem Vektor vorliegt.

25. Verfahren nach einem oder mehreren der Ansprüche 1 bis 25, wobei der rekombinante Vektor die Nukleotidsequenz SEQ ID NO. 9 hat.

26. Verfahren nach einem oder mehreren der Ansprüche 1 bis 26, wobei in der Zelle keine endogene γ-Sekretase Aktivität nachweisbar ist.

27. Verfahren nach einem oder mehreren der Ansprüche 1 bis 27, wobei die Zelle mit einer cDNA Bank co-transfiziert wird.

28. Verfahren nach Anspruch 28, wobei in der cDNA Bank, cDNA hergestellt aus einem humanen oder nicht humanen Gewebe oder humanen oder nicht humanen Zellen, vorliegt.

29. Verwendung eines Verfahrens nach einem oder mehreren der Ansprüche 27 bis 29 zur Identifizierung einer cDNA, die für eine γ-Sekretase kodiert, wobei
a) eine Zelle identifiziert wird, in der die Aktivität einer γ-Sekretase nachweisbar ist, und
b) aus dieser Zelle die cDNA, die für die γ-Sekretase kodiert, isoliert wird.

30. Verfahren zur Identifizierung von Substanzen, die die Aktivität der γ-Sekretase inhibieren, wobei das Verfahren die folgenden Verfahrensschritte enthält:
1. Herstellung eines transgenen nicht-humanen Organismus oder eine transgene Zelle, wobei der transgene nicht-humane Organismus oder die transgene Zelle ein Transgen enthält, das folgende Bestandteile hat:
a) eine erste Nukleotidsequenz, die für ein Protein, das die Aminosäuresequenz GAIIGLMVGGVVIATVIVITLVML (SEQ ID NO. 1) enthält, kodiert,
b) am 5'-Ende der ersten Nukleotidsequenz eine zweite Nukleotidsequenz, die für ein Signalpeptid kodiert, und
c) einen Promotor und
d) gegebenenfalls weitere nicht-kodierende und/oder kodierende Nukleotidsequenzen; und
ein Reporterplasmid enthält, das eine Proteinbindungsstelle, einen Minimalpromotor und ein Reportergen trägt und gegebenenfalls eine cDNA, die eine γ-Sekretase kodiert, wobei der transgene nicht-humane Organismus oder die transgene Zelle das Transgen und gegebenenfalls die durch die cDNA kodierte γ-Sekretase exprimiert
2. der transgene nicht-humane Organismus oder die transgene Zelle mit einer zu untersuchenden Substanz inkubiert wird und
3. das Fusionsprotein durch in der Zelle vorliegende γ-Sekretase innerhalb der Aminosäuresequenz SEQ ID NO. 1 gespalten wird, wodurch ein erstes Teilprotein, das die Aminosäuresequenz GAIIGLMVGGVV (SEQ CD NO. 2) enthält und ein zweites Teilprotein, das die Aminosäuresequenz VIVITLVML (SEQ ID NO. 3) enthält, gebildet werden und
4. die Menge an zweitem Teilprotein bestimmt und aus der Menge an gebildetem zweitem Teilprotein die Aktivität der γ-Sekretase bestimmt wird.

31. Verfahren zur Identifizierung von Substanzen, die die Aktivität der γ-Sekretase inhibieren, wobei
1. ein Transgen verwendet wird, das folgende Bestandteile enthält:
a) eine erste Nukleotidsequenz, die für ein Protein, das die Aminosäuresequenz GAIIGLMVGGVVIATVIVITLVML (SEQ ID NO. 1) enthält, kodiert,
b) am 5'-Ende der ersten Nukleotidsequenz eine zweite Nukleotidsequenz, die für ein Signalpeptid kodiert und
c) einen Promotor und
d) gegebenenfalls weitere kodierende und/oder nicht-kodierende Nukleotidsequenzen,
2. dieses Transgen und ein Reporterplasmid in eine Zelle eingebracht werden und das durch das Transgen kodierte Fusionsprotein und gegebenenfalls die durch die cDNA kodierte γ-Sekretase in Gegenwart einer zu untersuchenden Substanz exprimiert wird,
3. das Fusionsprotein durch in der Zelle vorliegende γ-Sekretase innerhalb der Aminosäuresequenz SEQ ID NO. 1 entweder
a) gespalten wird, wodurch ein erstes Teilprotein, das die Aminosäuresequenz GAIIGLMVGGW (SEQ ID NO. 2) enthält und ein zweites Teilprotein, das die Aminosäuresequenz VIVITLVML (SEQ ID NO. 3) enthält, gebildet werden, oder b) nicht gespalten wird, wodurch keine nachweisbare Menge an erstem und/oder zweitem Teilprotein gebildet wird,
4. die Menge an zweitem Teilprotein bestimmt wird.

## Claims

1. Process for the detection of the activity of γ-secretase, where
1. a transgene is used which encodes a fusion protein and contains the following constituents:
a) a first nucleotide sequence which codes for a protein which contains the amino acid sequence GAIIGLMVGGWIATVIVITLVML (SEQ ID NO. 1),
b) at the 5' end of the first nucleotide sequence, a second nucleotide sequence which codes for a signal peptide,
c) a promoter and,
d) if appropriate, further coding and/or noncoding nucleotide sequences;
2. this transgene is incorporated into a cell and the fusion protein is expressed;
3. the fusion protein is cleaved within the amino acid sequence SEQ ID NO. 1 by γ-secretase present in the cell, whereby a first partial protein, which contains the amino acid sequence GAIIGLMVGGW (SEQ ID NO. 2), and a second partial protein, which contains the amino acid sequence VIVITLVML (SEQ ID NO.3), are formed, and
4. the amount of second partial protein is determined and the activity of the Υ-secretase is determined from the amount of second partial protein formed.

2. Process according to one of Claims 1 and 2, where the first nucleotide sequence codes for an amyloid precursor protein (APP) or a part thereof.

3. Process according to one or more of Claims 1 to 3, where the first nuceotide sequence codes for a protein which contains the amino acid sequence (SEQ ID NO. 4).

4. Process according to one or more of Claims 1 to 4, where the second nucleotide sequence codes for the signal peptide of APP (SP).

5. Process according to one or more of claims 1 to 5, where the signal peptide has the amino acid sequence SEQ ID NO. 5.

6. Process according to one or more of Claims 1 to 6, where the promoter is a promoter for expression in mammalian cells, in C. elegans, in yeast or in Drosophila.

7. Process according to one or more of Claims 1 to 7, where the promoter is CMV, HSV TK, RSV, SV40, LTR, unc119, unc54, hsp16-2, G₀A1, sel-12, ADH 1, Gal1, MET3, MT, Ac5 or Ds47.

8. Process according to one or more of Claims 1 to 8, where the cell is a eukaryotic cell.

9. Process according to one or more of Claims 1 to 9, where the cell is an isolated, non-embryonic human cell.

10. Process according to one or more of Claims 1 to 9, where the cells is a nonhuman cell.

11. Process according to one or more of Claims 1 to 11, where the cell is an HeLa, 293, H4, SH-SY5Y, H9, Cos, CHO, N2A, SL-2 or Saccharomyces cerevisiae cell.

12. Process according to one or more of Claims 1 to 12, where the cell is a C. elegans cell.

13. Process according to Claim 12, where the cell is a constituent of a transgenic C. elegans.

14. Process according to Claim 11, where the cell is a yeast cell.

15. Process according to one or more of Claims 1 to 15, where the fusion protein has or contains the amino acid sequence SEQ ID NO. 6.

16. Process according to one or more of Claims 1 to 16, where the further coding nucleotide sequence is localized at the 3' end of the first nucleotide sequence.

17. Process according to one or more of Claims 1 to 17, where the further coding nucleotide sequence codes for a protein which is expressed as a fusion protein with the first partial protein and the second partial protein and can be used for the detection of the second partial protein.

18. Process according to one or more of Claims 1 to 18, where the further coding nucleotide sequence codes for a protein which contains a DNA-binding domain and a transcription-activating domain.

19. The process according to one or more of Claims 1 to 19, where the further coding nucleotide sequence codes for a protein which consists of a Gal4-binding domain and of the transcription-activating domain of VP16 (Gal4-VP16).

20. Process according to one or more of Claims 1 to 20, where the cell is cotransfected with a reporter plasmid, where the reporter plasmid contains a reporter gene under the control of a regulatable promoter.

21. Process according to Claim 21, where the regulatable promoter is activatable by the transcription-activating domain.

22. Process according to Claim 22, where the reporter plasmid encodes the reporter gene for EGFP (Enhanced Green Fluorescent Protein) and the regulatable promoter contains five Gal4 binding sites and a minimal promoter of HIV.

23. Process according to one or more of Claims 1 to 23, where the transgene has the nucleotide sequence SEQ ID NO. 8.

24. Process according to one or more of Claims 1 to 24, where the transgene is present in a vector.

25. Process according to one or more of Claims 1 to 25, where the recombinant vector has the nucleotide sequence SEQ ID NO. 9.

26. Process according to one or more of Claims 1 to 26, where no endogenous γ-secretase activity is detectable in the cell.

27. Process according to one or more of Claims 1 to 27, where the cell is cotransfected using a cDNA bank.

28. Process according to Claim 28, where cDNA prepared from a human or nonhuman tissue or human or nonhuman cells is present in the cDNA bank.

29. Use of a process according to one or more of Claims 27 to 29 for the identification of a cDNA which codes for a γ-secretase, where
a) a cell is identified in which the activity of a γ-secretase is detectable and
b) the cDNA which codes for the γ-secretase is isolated from this cell.

30. Process for the identification of substances which inhibit the activity of the γ-secretase, where the process comprises the following process steps:
1. production of a transgenic nonhuman organism or of a transgenic cell, where the transgenic nonhuman organism or the transgenic cell contains a transgene which has the following constituents:
a) a first nucleotide sequence which codes for a protein which contains the amino acid sequence GAIIGLMVGGVVIATVIVITLVML (SEQ ID NO. 1),
b) at the 5' end of the first nucleotide sequence, a second nucleotide sequence which codes for a signal peptide and
c) a promoter and,
d) if appropriate, further noncoding and/or coding nucleotide sequences;
contains a reporter plasmid which carries a protein binding site, a minimal promoter and a reporter gene and, if appropriate, a cDNA which encodes a γ-secretase,
where the transgenic nonhuman organism or the transgenic cell expresses the transgene and, if appropriate, the γ-secretase encoded by the cDNA;
2. the transgenic nonhuman organism or the transgenic cell is incubated with a substance to be investigated and
3. the fusion protein is cleaved within the amino acid sequence SEQ ID NO. 1 by γ-secretase present in the cell, whereby a first partial protein, which contains the amino acid sequence GAIIGLMVGGVV (SEQ ID NO. 2), and a second partial protein, which contains the amino acid sequence VIVITLVML (SEQ ID NO.3), are formed, and
4. the amount of second partial protein is determined and the activity of the Υ-secretase is determined from the amount of second partial protein formed.

31. Process for the identification of substances which inhibit the activity of the γ-secretase, where
1. a transgene is used which contains the following constituents:
a) a first nucleotide sequence which codes for a protein which contains the amino acid sequence GAIIGLMVGGVVIATVIVITLVML (SEQ ID NO. 1),
b) at the 5' end of the first nucleotide sequence, a second nucleotide sequence which codes for a signal peptide and
c) a promoter and,
d) if appropriate, further coding and/or noncoding nucleotide sequences;
2. this transgene and a reporter plasmid are incorporated into a cell and the fusion protein encoded by the transgene and, if appropriate, the γ-secretase encoded by the cDNA are expressed in the presence of a substance to be investigated,
3. the fusion protein is either
a) cleaved, whereby a first partial protein which contains the amino acid sequence GAIIGLMVGGW (SEQ ID NO. 2) and a second partial protein which contains the amino acid sequence VIVITLVML (SEQ ID NO. 3) are formed, or b) not cleaved, whereby no detectable amount of first and/or second partial protein is formed, within the amino acid sequence SEQ ID NO. 1 by γ-secretase present in the cell,
4. the amount of second partial protein is determined.

## Revendications

1. Procédé de mise en évidence de l'activité de la γ-secrétase, dans lequel:
1. on utilise un transgène qui code pour une protéine de fusion et contient les constituants suivants:
a) une première séquence de nucléotides qui code pour une protéine contenant la séquence d'acides aminés GAIIGLMVGGVVIATVIVITLVML (SEQ ID n° 1),
b) sur l'extrémité 5' de la première séquence de nucléotides, une deuxième séquence de nucléotides qui code pour un peptide signal,
c) un promoteur et
d) le cas échéant, d'autres séquences de nucléotides codantes et/ou non codantes,
2. ce transgène est introduit dans une cellule et la protéine de fusion est exprimée,
3. la protéine de fusion est clivée par la γ-secrétase présente dans la séquence d'acides aminés SEQ ID n° 1, suite à quoi il se forme une première protéine partielle qui contient la séquence d'acides aminés GAIIGLMVGGVV (SEQ ID n° 2) et une deuxième protéine partielle qui contient la séquence d'acides aminés VIVITLVML (SEQ ID n° 3) et
4. la quantité de deuxième protéine partielle est déterminée, et l'activité de la γ-secrétase est déterminée sur la base de la quantité de deuxième protéine partielle formée.

2. Procédé suivant l'une quelconque des revendications 1 ou 2, dans lequel la première séquence de nucléotides code pour une protéine précurseur d'amyloïde (APP) ou une partie de celle-ci.

3. Procédé suivant une ou plusieurs des revendications 1 à 3, dans lequel la première séquence de nucléotides code pour une protéine qui contient la séquence d'acides aminés (SEQ ID n° 4).

4. Procédé suivant une ou plusieurs des revendications 1 à 4, dans lequel la deuxième séquence de nucléotides code pour le peptide signal de l'APP (SP).

5. Procédé suivant une ou plusieurs des revendications 1 à 5, dans lequel le peptide signal contient la séquence d'acides aminés SEQ ID n° 5.

6. Procédé suivant une ou plusieurs des revendications 1 à 6, dans lequel le promoteur est un promoteur d'expression dans des cellules de mammifères, dans C. elegans, dans des levures ou dans des drosophiles.

7. Procédé suivant une ou plusieurs des revendications 1 à 7, dans lequel le promoteur est un CMV, HSV TK, RSV, SV40, LTR, unc119, unc54, hsp16-2, G0A1, sel-12, ADHI, Gall, MET3, MT, Ac5 ou Ds47.

8. Procédé suivant une ou plusieurs des revendications 1 à 8, dans lequel la cellule est une cellule eucaryote.

9. Procédé suivant une ou plusieurs des revendications 1 à 9, dans lequel la cellule est une cellule humaine isolée non embryonnaire.

10. Procédé suivant une ou plusieurs des revendications 1 à 10, dans lequel la cellule est une cellule non humaine.

11. Procédé suivant une ou plusieurs des revendications 1 à 11, dans lequel la cellule est une cellule HeLa, 293, H4, SH-SY5Y, H9, Cos, CHO, N2A, SL-2 ou de Saccharomyces cerevisiae.

12. Procédé suivant une ou plusieurs des revendications 1 à 12, dans lequel la cellule est une cellule de C. elegans.

13. Procédé suivant la revendication 12, dans lequel la cellule est un constituant d'un C. elegans transgénique.

14. Procédé suivant la revendication 11, dans lequel la cellule est une cellule de levure.

15. Procédé suivant une ou plusieurs des revendications 1 à 15, dans lequel la protéine de fusion possède ou contient la séquence d'acides aminés SEQ ID n° 6.

16. Procédé suivant une ou plusieurs des revendications 1 à 16, dans lequel l'autre séquence de nucléotides codante est localisée à l'extrémité 3' de la première séquence de nucléotides.

17. Procédé suivant une ou plusieurs des revendications 1 à 17, dans lequel l'autre séquence codante de nucléotides code pour une protéine qui est exprimée en tant que protéine de fusion avec la première protéine partielle et la deuxième protéine partielle et peut être utilisée pour la détection de la deuxième protéine partielle.

18. Procédé suivant une ou plusieurs des revendications 1 à 18, dans lequel l'autre séquence de nucléotides codante code pour une protéine contenant un domaine de liaison d'ADN et un domaine d'activation de transcription.

19. Procédé suivant une ou plusieurs des revendications 1 à 19, dans lequel l'autre séquence de nucléotides codante code pour une protéine qui consiste en un domaine lié à Gal4 et en le domaine activateur de transcription de VP16 (Gal4-VP16).

20. Procédé suivant une ou plusieurs des revendications 1 à 20, dans lequel la cellule est co-transfectée avec un plasmide reporter, le plasmide reporter contenant un gène reporter sous le contrôle d'un promoteur régulable.

21. Procédé suivant la revendication 21, dans lequel le promoteur régulable peut être activé par les domaines d'activation de la transcription.

22. Procédé suivant la revendication 22, dans lequel le plasmide reporter code pour le gène reporter EGFP (Enhanced Green Fluorescent Protein, protéine de fluorescence verte amplifiée) et le promoteur régulable contient cinq sites de liaison de Gal4 et un promoteur minimal de VIH.

23. Procédé suivant une ou plusieurs des revendications 1 à 23, dans lequel le transgène possède la séquence de nucléotides SEQ ID n° 8.

24. Procédé suivant une ou plusieurs des revendications 1 à 24, dans lequel le transgène se trouve dans un vecteur.

25. Procédé suivant une ou plusieurs des revendications 1 à 25, dans lequel le vecteur recombinant possède la séquence de nucléotides SEQ ID n° 9.

26. Procédé suivant une ou plusieurs des revendications 1 à 26, dans lequel aucune activité endogène de la γ-secrétase ne peut être mise en évidence dans la cellule.

27. Procédé suivant une ou plusieurs des revendications 1 à 27, dans lequel la cellule est transfectée avec une banque d'ADNc.

28. Procédé suivant la revendication 28, dans lequel la banque d'ADNc contient de l'ADNc préparé à partir de tissus humains ou non humains ou de cellules humaines ou non humaines.

29. Utilisation d'un procédé suivant une ou plusieurs des revendications 27 à 29 pour l'identification d'un ADNc codant pour une γ-secrétase, dans lequel:
a) on identifie une cellule dans laquelle l'activité d'une γ-secrétase peut être mise en évidence, et
b) on isole à partir de cette cellule l'ADNc qui code pour la γ-secrétase.

30. Procédé d'identification de substances inhibant l'activité de la γ-secrétase, le procédé comprenant les étapes suivantes :
1. Préparation d'un organisme transgénique non humain ou d'une cellule transgénique, où l'organisme transgénique non humain ou la cellule transgénique contient un transgène possédant les constituants suivants :
a) une première séquence de nucléotides qui code pour une protéine contenant la séquence d'acides aminés GAIIGLMVGGVVIATVIVITLVML (SEQ ID n° 1),
b) sur l'extrémité 5' de la première séquence de nucléotides, une deuxième séquence de nucléotides qui code pour un peptide signal,
c) un promoteur et
d) le cas échéant, d'autres séquences de nucléotides non codantes et/ou codantes, et
un plasmide reporter qui porte un site de liaison de protéine, un promoteur minimal et un gène reporter et le cas échéant un ADNc codant pour une γ-secrétase, où l'organisme transgénique non humain ou la cellule transgénique exprime le transgène et le cas échéant la γ-secrétase pour laquelle code l'ADNc,
2. l'organisme transgénique non humain ou la cellule transgénique est incubée avec une substance à étudier et
3. la protéine de fusion est clivée par la γ-secrétase présente dans la séquence d'acides aminés SEQ ID n° 1, suite à quoi il se forme une première protéine partielle qui contient la séquence d'acides aminés GAIIGLMVGGVV (SEQ ID n°2) et une deuxième protéine partielle qui contient la séquence d'acides aminés VIVITLVML (SEQ ID n° 3) et
4. la quantité de deuxième protéine partielle est déterminée, et l'activité de la γ-secrétase est déterminée sur la base de la quantité de deuxième protéine partielle formée.

31. Procédé d'identification de substances inhibant l'activité de la γ-secrétase, dans lequel :
1. on utilise un transgène qui code pour une protéine de fusion et contient les constituants suivants :
a) une première séquence de nucléotides qui code pour une protéine contenant la séquence d'acides aminés GAIIGLMVGGVVIATVIVITLVML (SEQ ID n° 1),
b) sur l'extrémité 5' de la première séquence de nucléotides, une deuxième séquence de nucléotides qui code pour un peptide signal,
c) un promoteur et
d) le cas échéant, d'autres séquences de nucléotides codantes et/ou non codantes,
2. ce transgène et un plasmide reporter sont introduits dans une cellule et on exprime la protéine de fusion pour laquelle code le transgène et le cas échéant la γ-secrétase pour laquelle code l'ADNc en présence d'une substance à étudier,
3. la protéine de fusion, soit a) est clivée par la γ-secrétase présente dans la séquence d'acides aminés SEQ ID n° 1, suite à quoi il se forme une première protéine partielle qui contient la séquence d'acides aminés GAIIGLMVGGVV (SEQ ID n° 2) et une deuxième protéine partielle qui contient la séquence d'acides aminés VIVITLVML (SEQ ID n° 3), soit b) n'est pas clivée, auquel cas il ne se forme pas de quantité décelable de première et/ou de deuxième protéine partielle,
4. on détermine la quantité de deuxième protéine partielle.
